(19) Europäisches Patentamt / European Patent Office / Office européen des brevets

(11) **EP 2 553 119 B1**

(12) **EUROPEAN PATENT SPECIFICATION**

(45) Date of publication and mention of the grant of the patent:
**30.03.2016 Bulletin 2016/13**

(21) Application number: **11711550.1**

(22) Date of filing: **30.03.2011**

(51) Int Cl.:
*C12Q 1/68* $^{(2006.01)}$     *G01N 33/574* $^{(2006.01)}$

(86) International application number:
**PCT/EP2011/054915**

(87) International publication number:
**WO 2011/121028 (06.10.2011 Gazette 2011/40)**

(54) **ALGORITHM FOR PREDICTION OF BENEFIT FROM ADDITION OF TAXANE TO STANDARD CHEMOTHERAPY IN PATIENTS WITH BREAST CANCER**

ALGORITHMUS ZUR VORHERSAGE DER VORTEILHAFTIGKEIT EINER ZUGABE VON TAXAN ZUR STANDARDCHEMOTHERAPIE BEI PATIENTEN MIT BRUSTKREBS

ALGORITHME POUR LA PRÉDICTION DU BÉNÉFICE DE L'AJOUT DE TAXANE À UNE CHIMIOTHÉRAPIE STANDARD CHEZ DES PATIENTS ATTEINTS D'UN CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2010 EP 10158394**

(43) Date of publication of application:
**06.02.2013 Bulletin 2013/06**

(73) Proprietors:
• **Wirtz, Ralph**
  **50677 Köln (DE)**
• **BioNTech Diagnostics GmbH**
  **55131 Mainz (DE)**

(72) Inventors:
• **FOUNTZILAS, George**
  **GR-55236 Panorama**
  **Thessaloniki (GR)**
• **GEHRMANN, Mathias**
  **51375 Leverkusen (DE)**
• **KRONENWETT, Ralf**
  **51063 Cologne (DE)**
• **STROPP, Udo**
  **42781 Haan (DE)**
• **WEBER, Karsten**
  **51379 Leverkusen (DE)**
• **WIRTZ, Ralph**
  **50667 Cologne (DE)**

(74) Representative: **Schnappauf, Georg et al**
  **ZSP Patentanwälte PartG mbB**
  **Radlkoferstrasse 2**
  **81373 München (DE)**

(56) References cited:
**WO-A1-2009/050014**    **WO-A1-2010/003773**
**WO-A1-2010/076322**    **US-A1- 2003 124 128**

• **TANG SHOU-CHING: "Predictive markers of tubulin-targeting agents in breast cancer.", CLINICAL BREAST CANCER MAR 2008 LNKD-PUBMED:18637403, vol. 8 Suppl 2, March 2008 (2008-03), pages S79-S84, XP002636994, ISSN: 1526-8209**
• **VON MINCKWITZ G ET AL: "Validated 3 Gene Signature Predicts Response to Neo-Adjuvant Chemotherapy in Luminal Breast Cancer - Results from GeparTrio and GeparQuattro", CANCER RESEARCH, AMERICAN ASSOCIATION FOR CANCER REREARCH, US, vol. 69, no. 24, suppl, 1 December 2009 (2009-12-01), page 635S, XP009131681, ISSN: 0008-5472**

SEQUENCE LISTING

<110> Siemens Healthcare Diagnostics Inc.

<120> Algorithm for prediction of benefit from addition of taxane to standard chemotherapy in patients with breast cancer

<130> 200922916

<160> 27

<170> PatentIn version 3.3

<210> 1
<211> 19
<212> DNA
<213> Artificial

<220>
<223> oligonucleotide

<400> 1
cggggcagtt gcagtggtg         19

<210> 2
<211> 30
<212> DNA

<213> Artificial

<220>
<223> oligonucleotide

<400> 2
agtcaactgg aactcaaaaa cctgcacgtt 30

<210> 3
<211> 22
<212> DNA
<213> Artificial

<220>
<223> oligonucleotide

<400> 3
tcggtctgct ggtctccgct cc        22

<210> 4
<211> 15
<212> DNA
<213> Artificial

<220>
<223> oligonucleotide

<400> 4
cgaccaagga aaact        15

<210> 5
<211> 27
<212> DNA
<213> Artificial

<220>
<223> oligonucleotide

<400> 5
ttgagtcatc tccagcatcc gaggaaa        27

<210> 6
<211> 17
<212> DNA
<213> Artificial

<220>
<223> oligonucleotide

<400> 6
ctgtttagcg acttcag        17

<210> 7
<211> 18
<212> DNA
<213> Artificial

<220>

<223> oligonucleotide

<400> 7
tggtcatgga gtagaagg          18

<210> 8
<211> 23
<212> DNA
<213> Artificial

<220>
<223> oligonucleotide

<400> 8
agaagcgggc ctcaccatcc aag          23

<210> 9
<211> 29
<212> DNA
<213> Artificial

<220>
<223> oligonucleotide

<400> 9
ccactaaccg acttggctgc ttcctctag          29

<210> 10
<211> 21
<212> DNA
<213> Artificial

<220>
<223> oligonucleotide

<400> 10
cctacctcct cacggctctt c          21

<210> 11
<211> 21
<212> DNA
<213> Artificial

<220>
<223> oligonucleotide

<400> 11
ctacaatgag cagcgcatca g          21

<210> 12
<211> 26
<212> DNA
<213> Artificial

<220>
<223> oligonucleotide

<400> 12

ctgattgaag atcattttca acatca     26

<210> 13
<211> 19
<212> DNA
<213> Artificial

<220>
<223> oligonucleotide

<400> 13
cagccttctc agccaaacg     19

<210> 14
<211> 22
<212> DNA
<213> Artificial

<220>
<223> oligonucleotide

<400> 14
ccacagatgt catggaatcc at 22

<210> 15
<211> 29
<212> DNA
<213> Artificial

<220>
<223> oligonucleotide

<400> 15
gactggacat atcacctcta cttaaatcc     29

<210> 16
<211> 28
<212> DNA
<213> Artificial

<220>
<223> oligonucleotide

<400> 16
gattgaagat actccatctt ctaaacca     28

<210> 17
<211> 23
<212> DNA
<213> Artificial

<220>
<223> oligonucleotide

<400> 17
ggccttccat cttaccttca aaa     23

<210> 18

<211> 24
<212> DNA
<213> Artificial

<220>
<223> oligonucleotide

<400> 18
aaagggaacg gtgaagtact aagc          24

<210> 19
<211> 26
<212> DNA
<213> Artificial

<220>
<223> oligonucleotide

<400> 19
aatgtctgga gatggttctg ttaatg 26

<210> 20
<211> 26
<212> DNA
<213> Artificial

<220>
<223> oligonucleotide

<400> 20
ggagatttct gtctaggcca tctatg          26

<210> 21
<211> 20
<212> DNA
<213> Artificial

<220>
<223> oligonucleotide

<400> 21
gacggccatt cagcttatgg          20

<210> 22
<211> 26
<212> DNA
<213> Artificial

<220>
<223> oligonucleotide

<400> 22
caaatcactg caattctcat ggtagt          26

<210> 23
<211> 20
<212> DNA
<213> Artificial

```
<220>
<223> oligonucleotide

<400> 23
tgcctggctc aggaagattt        20

<210> 24
<211> 23
<212> DNA
<213> Artificial

<220>
<223> oligonucleotide

<400> 24
tgtctttctg gcctatggac tca        23

<210> 25
<211> 25
<212> DNA
<213> Artificial

<220>
<223> oligonucleotide

<400> 25
ttcagttgga gaccctgtag tatcg        25

<210> 26
<211> 24
<212> DNA
<213> Artificial

<220>
<223> oligonucleotide

<400> 26
ttttgcaaca gtctgagtgc tctt        24
<210> 27
<211> 20
<212> DNA
<213> Artificial

<220>
<223> oligonucleotide

<400> 27
aactgggcac caatcatgct        20
```

**Description**

In cancer therapy it is still a challenge to find the optimal therapy for a patient. For example breast Cancer is the leading cause of death in women between ages of 35 - 55. Worldwide, there are over 3 million women living with breast cancer. OECD (Organization for Economic Cooperation & Development) estimates on a worldwide basis 500,000 new cases of breast cancer are diagnosed each year. One out of ten women will face the diagnosis breast cancer at some point during her lifetime.

According to today's therapy guidelines and current medical practice, the selection of a specific therapeutic intervention is mainly based on histology, grading, staging and hormonal status of the patient. Several studies have shown that adjuvant chemotherapy in patients with operable clinically high risk breast cancer is able to reduce the annual odds of recurrence and death. One of the first adjuvant treatment regimens was a combination of cyclophosphamide, methotrexate and 5-fluoruracil (CMF). Subsequently, anthracyclines were introduced in the adjuvant breast cancer therapy resulting in an improvement of 5 years disease-free survival (DFS) of 3% in comparison with CMF. The addition of taxanes to anthracyclines resulted in a further increase of 5 years DFS of 4-7%. However, taxane-containing regimens are usually more toxic than conventional anthracycline-containing regimens resulting in a benefit only for a small percentage of patients. Currently, there are no reliable predictive markers to identify the subgroup of patients who benefit from taxanes and many aspects of a patient's specific type of tumor are currently not assessed - preventing true patient-tailored treatment.

Thus several open issues in current therapeutic strategies remain. One point is the practice of significant over-treatment of patients; it is well known from past clinical trials that 70% of breast cancer patients with early stage disease do not need any treatment beyond surgery. While about 90% of all early stage cancer patients receive chemotherapy exposing them to significant treatment side effects, approximately 30% of patients with early stage breast cancer relapse. On the other hand, one fourth of clinically high risk patients suffer from distant metastasis during five years despite conventional cytotoxic chemotherapy. Those patients are undertreated and need additional or alternative therapies. Finally, one of the most open questions in current breast cancer therapy is which patients have a benefit from addition of taxanes to conventional chemotherapy.

As such, there is a significant medical need to develop diagnostic assays that identify low risk patients for directed therapy. For patients with medium or high risk assessment, there is a need to pinpoint therapeutic regimens tailored to the specific cancer to assure optimal success. A test that can predict if patients with high-risk tumors can be safely treated with conventional anthracycline-based chemotherapy or require the addition of taxanes has a high medical need.

Curing breast cancer patients is still a challenge for the treating oncologist as the diagnosis relies in most cases on clinical and pathological data like age, menopausal status, hormonal status, grading, and general constitution of the patient and some molecular markers like Her2/neu, p53, and others. Recent studies could show that patients with so called triple negative breast cancer have a benefit from taxanes. Unfortunately, until recently, there was no test in the market for prognosis or therapy prediction that come up with a more elaborated recommendation for the treating oncologist whether and how to treat patients. Predictive assays are until now not in the market but two for prognosis: Genomic Health's OncotypeDXTM and Agendia's MammaprintTM. In 2007, the company Agendia got FDA approval for their MammaprintTM microarray assay that can predict with the help of 70 informative genes and a great number of housekeeping genes the prognosis of breast cancer patients from fresh tissue (shipped in RNALaterTM). Genomic Health works with formalin-fixed and paraffin-embedded tumor tissues and uses 21 genes for their prognosis prediction, presented as a risk score. Additionally, Genomic Health could show that their OncotypeDXTM is also predictive of CMF chemotherapy benefit in node-negative, ER positive patients. Genomic Health could also show that their recurrence score in combination with further candidate genes predicts taxane benefit.

Objective of the invention

It is an objective of the invention to provide a method for the prediction of outcome of cancer relying on a limited number of markers.

It is a further objective of the invention to provide a method for identification of patients who have a benefit from the addition of a taxane to standard adjuvant chemotherapy.

Definitions

Unless defined otherwise, technical and scientific terms used herein have the same meaning as commonly understood by one of ordinary skill in the art.

The term "cancer" is not limited to any stage, grade, histomorphological feature, aggressivity, or malignancy of an affected tissue or cell aggregation.

The term "predicting an outcome" of a disease, as used herein, is meant to include both a prediction of an outcome of a patient undergoing a given therapy and a prognosis of a patient who is not treated. The term "predicting an outcome" may, in particular, relate to the risk of a patient developing metastasis, local recurrence or death.

The term "prediction", as used herein, relates to an individual assessment of the malignancy of a tumor, or to the expected survival rate (OAS, overall survival or DFS, disease free survival) of a patient, if the tumor is treated with a given therapy. In contrast thereto, the term "prognosis" relates to an individual assessment of the malignancy of a tumor, or to the expected survival rate (OAS, overall survival or DFS, disease free survival) of a patient, if the tumor remains untreated.

An "outcome" as used herein, is a defined condition attained in the course of the disease. This disease outcome may e.g. be a clinical condition such as "recurrence of disease", "development of metastasis", "development of nodal metastasis", "development of distant metastasis", "survival", "death", "tumor remission rate", a disease stage or grade or the like.

A "risk" is understood to be a probability of a subject or a patient to develop or arrive at a certain disease outcome. The term "risk" is not meant to carry any positive or negative connotation with regard to a patient's wellbeing but merely refers to a probability or likelihood of an occurrence or development of a given condition.

A "benefit" from a given therapy is an improvement in health or wellbeing that can be observed in patients under said therapy, but isn't observed in patients not receiving this therapy. Non-limiting examples commonly used in oncology to gauge a benefit from therapy are survival, disease free survival, metastasis free survival, disappearance of metastasis, tumor regression, and tumor remission.

The term "clinical data" relates to the entirety of available data and information concerning the health status of a patient including, but not limited to, age, sex, weight, menopausal/hormonal status, etiopathology data, anamnesis data, data obtained by in vitro diagnostic methods such as histopathology, blood or urine tests, data obtained by imaging methods, such as x-ray, computed tomography, MRI, PET, spect, ultrasound, electrophysiological data, genetic analysis, gene expression analysis, biopsy evaluation, intraoperative findings.

The term "node positive", "diagnosed as node positive", "node involvement" or "lymph node involvement" means a patient having previously been diagnosed with lymph node metastasis. It shall encompass both draining lymph node, near lymph node, and distant lymph node metastasis. This previous diagnosis itself shall not form part of the inventive method. Rather it is a precondition for selecting patients whose samples may be used for one embodiment of the present invention. This previous diagnosis may have been arrived at by any suitable method known in the art, including, but not limited to lymph node removal and pathological analysis, biopsy analysis, in-vitro analysis of biomarkers indicative for metastasis, imaging methods (e.g. computed tomography, X-ray, magnetic resonance imaging, ultrasound), and intraoperative findings.

A "biological sample" is a sample which is derived from or has been in contact with a biological organism. Examples for biological samples are: cells, tissue, body fluids, lavage fluid, smear samples, biopsy specimens, blood, urine, saliva, sputum, plasma, serum, cell culture supernatant, and others. A "tumor sample" is a biological sample containing tumor cells, whether intact or degraded.

A "gene" is a set of segments of nucleic acid that contains the information necessary to produce a functional RNA product. A "gene product" is a biological molecule produced through transcription or expression of a gene, e.g. an mRNA or the translated protein.

An "mRNA" is the transcribed product of a gene and shall have the ordinary meaning understood by a person skilled in the art. A "molecule derived from an mRNA" is a molecule which is chemically or enzymatically obtained from an mRNA template, such as cDNA.

The term "expression level" refers to a determined level of gene expression. This may be a determined level of gene expression as an absolute value or compared to a reference gene (e.g. a housekeeping gene) or to a computed average expression value (e.g. in DNA chip analysis) or to another informative gene without the use of a reference sample. The expression level of a gene may be measured directly, e.g. by obtaining a signal wherein the signal strength is correlated to the amount of mRNA transcripts of that gene or it may be obtained indirectly at a protein level, e.g. by immunohistochemistry, CISH, ELISA or RIA methods. The expression level may also be obtained by way of a competitive reaction to a reference sample. An expression value which is determined by measuring some physical parameter in an assay, e.g. fluorescence emission, may be assigned a numerical value which may be used for further processing of information.

A "reference pattern of expression levels", as used herein, shall be understood as being any pattern of expression levels that can be used for the comparison to another pattern of expression levels. In a preferred embodiment, a reference, pattern of expression levels is, e.g., an average pattern of expression levels observed in a group of healthy individuals, diseased individuals, or diseased individuals having received a particular type of therapy, serving as a reference group.

The term "mathematically combining expression levels", as used herein, shall be understood as deriving a numeric value from a determined expression level of a gene and applying an algorithm to obtain a combined numerical value or combined score.

An "algorithm" is a process that performs some sequence of operations to process an information.

A "discriminant function" is a function of a set of variables used to classify an object or event. A discriminant function thus allows classification of a patient, sample or event into a category or a plurality of categories according to data or parameters available from said patient, sample or event. Such classification is a standard instrument of statistical analysis well known to the skilled person. E.g. a patient may be classified as "high risk" or "low risk", "high probability of metastasis" or "low probability of metastasis", "in need of treatment" or "not in need of treatment" according to data obtained from said patient, sample or event. Classification is not limited to "high vs. low", but may be performed into a plurality categories, grading or the like. Classification shall also be understood in a wider sense as a discriminating score, where e.g. a higher score represents a higher likelihood of distant metastasis, e.g. the (overall) risk of a distant metastasis. Examples for discriminant functions which allow a classification include, but are not limited to functions defined by support vector machines (SVM), k-nearest neighbors (kNN), (naive) Bayes models, linear regression models or piecewise defined functions such as, for example, in subgroup discovery, in decision trees, in logical analysis of data (LAD) and the like. In a wider sense, continuous score values of mathematical methods or algorithms, such as correlation coefficients, projections, support vector machine scores, other similarity-based methods, combinations of these and the like are examples for illustrative purpose.

The term "therapy modality", "therapy mode", "regimen" or "chemo regimen" as well as "therapy regimen" refers to a timely sequential or simultaneous administration of antitumor, and/or anti vascular, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in the dose of the single agent, timeframe of application and frequency of administration within a defined therapy window. Currently various combinations of various drugs and/or physical methods, and various schedules are under investigation.

The term "cytotoxic treatment" refers to various treatment modalities affecting cell proliferation and/or survival. The treatment may include administration of alkylating agents, antimetabolites, anthracyclines, plant alkaloids, topoisomerase inhibitors, and other antitumour agents, including monoclonal antibodies and kinase inhibitors. In particular, the cytotoxic treatment may relate to a treatment comprising microtubule-stabilizing drugs such as taxanes or epothilones. Taxanes are plant alkaloids which block cell division by preventing microtubule function. The prototype taxane is the natural product paclitaxel, originally known as Taxol and first derived from the bark of the Pacific Yew tree. Docetaxel is a semi-synthetic analogue of paclitaxel. Taxanes enhance stability of microtubules, preventing the separation of chromosomes during anaphase. To improve pharmacokinetics and cellular uptake taxanes can be bound to delivery vehicles such as for example albumin (abraxane). Epothilones such as for example Ixabepilone stabilize the microtubules, have the same biological effects and target the same binding site at the microtubule as taxol. However, the chemical structure is different.

A "taxane-based" treatment or therapy is a treatment or therapy comprising taxol or therapeutically effective derivatives thereof. The principal mechanism of the taxane class of drugs is the disruption of microtubule function.

A "microtubule stabilizing agent-based" treatment or therapy is a treatment or therapy comprising taxol or therapeutically effective derivatives thereof, epothilones or therapeutically effective derivatives thereof or other microtubule stabilizing cytotoxic drugs.

The term "hybridization-based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described above. When it comes to the determination of expression levels, hybridization based methods may for example be used to determine the amount of mRNA for a given gene.

An oligonucleotide capable of specifically binding sequences a gene or fragments thereof relates to an oligonucleotide which specifically hybridizes to a gene or gene product, such as the gene's mRNA or cDNA or to a fragment thereof. To specifically detect the gene or gene product, it is not necessary to detect the entire gene sequence. A fragment of about 20-150 bases will contain enough sequence specific information to allow specific hybridization.

The term "a PCR based method" as used herein refers to methods comprising a polymerase chain reaction (PCR). This is a method of exponentially amplifying nucleic acids, e.g. DNA by enzymatic replication in vitro. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR).

Moreover, PCR-based methods comprise e.g. real time PCR, and, particularly suited for the analysis of expression levels, kinetic or quantitative PCR (qPCR).

The term "Quantitative PCR" (qPCR)" refers to any type of a PCR method which allows the quantification of the template in a sample. Quantitative real-time PCR comprise different techniques of performance or product detection as for example the TaqMan technique or the LightCycler technique. The TaqMan technique, for examples, uses a dual-labelled fluorogenic probe. The TaqMan real-time PCR measures accumulation of a product via the fluorophore during the exponential stages of the PCR, rather than at the end point as in conventional PCR. The exponential increase of the product is used to determine the threshold cycle, CT, i.e. the number of PCR cycles at which a significant exponential increase in fluorescence is detected, and which is directly correlated with the number of copies of DNA template present in the reaction. The set up of the reaction is very similar to a conventional PCR, but is carried out in a real-time thermal cycler that allows measurement of fluorescent molecules in the PCR tubes. Different from regular PCR, in TaqMan real-time PCR a probe is added to the reaction, i.e., a single-stranded oligonucleotide complementary to a segment of 20-60 nucleotides within the DNA template and located between the two primers. A fluorescent reporter or fluorophore (e.g., 6-carboxyfluorescein, acronym: FAM, or tetrachlorofluorescin, acronym: TET) and quencher (e.g., tetramethylrhodamine, acronym: TAMRA, of dihydrocyclopyrroloindole tripeptide "minor groove binder", acronym: MGB) are covalently attached to the 5' and 3' ends of the probe , respectively[2]. The close proximity between fluorophore and quencher attached to the probe inhibits fluorescence from the fluorophore. During PCR, as DNA synthesis commences, the 5' to 3' exonuclease activity of the Taq polymerase degrades that proportion of the probe that has annealed to the template (Hence its name: Taq polymerase + PacMan). Degradation of the probe releases the fluorophore from it and breaks the close proximity to the quencher, thus relieving the quenching effect and allowing fluorescence of the fluorophore. Hence, fluorescence detected in the real-time PCR thermal cycler is directly proportional to the fluorophore released and the amount of DNA template present in the PCR.

By "array" or "matrix" an arrangement of addressable locations or "addresses" on a device is meant. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, nucleotide analogues, polynucleotides, polymers of nucleotide analogues, morpholinos or larger portions of genes. The nucleic acid and/or analogue on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about $100/cm^2$, and preferably at least about $1000/cm^2$.

Summary of the invention

We describe herein methods to identify a patient subcohort deriving a particular benefit from Taxane treatment and a subcohort having no benefit or even an adverse effect regarding outcome. The algorithms were identified in patients from a randomized clinical taxane trial with patients suffering from high-risk breast cancer. Outcome refers to getting a distant metastasis or relapse within 5 years (high risk) and overall survival.

This disclosure focuses on a predictive cancer test that will help the oncologist to identify patients who will have a benefit from addition of taxanes to standard adjuvant chemotherapy and thus will help to make decisions on therapeutic regimens.

It was found that the gene expression of both AKR1C3 and SPP1, and more generally of any of the marker genes AKR1C3, MAP4, SPP1, CXCL9, PTGER3, and VEGFC is generally indicative for benefit from taxane-based therapy in patients. For AKR1C3, MAP4, SPP1, PTGER3, and VEGFC a high expression and for CXCL9 a low expression generally indicates an increased likelihood of benefit from taxane-based therapy, wherein an increased level of gene expression of AKR1C3 and SPP1 in comparison to a reference pattern of expression is indicative of a benefit from microtubule stabilizing agent-based cytotoxic chemotherapy.

The invention relates to a method for predicting an outcome of cancer in a patient, said method comprising:

(a) determining in a biological sample from said patient the expression levels of the genes AKR1C3, and SPP1,
(b) mathematically combining said expression levels to yield a combined score, wherein said combined score is indicative of benefit from cytotoxic chemotherapy comprising a microtubule stabilizing agent-based therapy of said patient.

The mathematical combination comprises the use of an algorithm to determine the combined score, in particular the use of a discriminant function. Such algorithms may comprise the use of linear combinations, averages, weighted averages, sums, differences, products and/or linear and nonlinear functions to arrive at the combined score.

According to an aspect of the invention further the expression levels of at least one of the genes PTGER3, CXCL9, MAP4 and/or VEGFC are determined.

According to an aspect of the invention one, two or more thresholds are determined for said combined score and discriminated into (1) "predicted benefit" and "predicted non-benefit", (2) "predicted benefit" and "predicted adverse effect", (3) "predicted benefit", "predicted indifferent effect" and "predicted adverse effect", or more risk groups with different probabilities of benefit by applying the threshold on the combined score.

According to an aspect of the invention said prediction of outcome is the determination of the risk of recurrence of cancer in said patient within 5 to 10 years or the risk of developing distant metastasis in a similar time horizon, or the prediction of death or of death after recurrence within 5 to 10 years after surgical removal of the tumor.

According to an aspect of the invention said prediction of outcome is a classification of said patient as belonging into one of two or three distinct classes, said classes corresponding to a "high risk " class, (an "intermediate risk" class) and a "low risk" class. In this context "risk" merely refers to the likelihood of an event occurring and is not intend to carry a positive or negative connotation. E.g. the "high risk class" may relate to a high risk of developing metastasis under a given therapy or it may also relate e.g. to a high likelihood of benefit from a given therapy.

According to an aspect of the invention said cancer is breast cancer. The marker genes described in this invention are not breast cancer specific genes, but generally cancer-relevant genes or genes relevant to the therapeutic mechanism of microtubule stabilizing drugs. It can therefore be expected that the methods of the invention are also predictive in other cancers, in which taxane-based therapy is commonly administered, such as lung cancer, head-and-neck cancer, ovarian cancer und prostate cancer.

According to an aspect of the invention said determination of expression levels is in a formalin-fixed paraffin embedded sample, in a fixed sample, in a fresh sample or in a fresh-frozen sample.

According to an aspect of the invention the method may be applied to a patient who has previously received treatment by surgery and cytotoxic chemotherapy.

According to an aspect of the invention the microtubule stabilizing agent-based cytotoxic chemotherapy is a taxan-based cytotoxic chemotherapy.

According to an aspect of the invention said step of mathematically combining comprises a step of applying an algorithm to values representative of an expression level of a given gene. Preferred algorithms are described below.

According to an aspect of the invention said algorithm is a linear combination of said values representative of an expression level of a given gene.

According to an aspect of the invention a value for a representative of an expression level of a given gene is multiplied with a coefficient.

According to an aspect of the disclosure a high expression level of any given gene of the genes AKR1C3, MAP4, SPP1, PTGER3, and VEGFC or low expression of the gene CXCL9 is indicative of benefit from cytotoxic chemotherapy comprising microtubule-stabilizing drugs.

According to an aspect of the invention a high combined score is indicative of benefit from microtubule stabilizing agent-based. The combined score can be transformed to a given scale in an additional step. Said transformation may be linear or non-linear, continuous or discontinuous, bounded or unbounded, monotonic or non-monotonic.

According to an aspect of the invention wherein the expression levels are determined as mRNA expression levels by at least one of a hybridization based method, a PCR based method, an array based method.

Further disclosed is a kit for performing a method of any of claims 1 to 13, said kit comprising a set of oligonucleotides capable of specifically binding sequences of the genes AKR1C3, and SPP1 or fragments thereof.

The invention further relates to the use of a kit for performing a method of any of claims 1 to 13, said kit comprising a set of oligonucleotides capable of specifically binding sequences of the genes AKR1C3, and SPP1 and, optionally, MAP4, CXCL9, PTGER3 and/or VEGFC, or fragments thereof.

The invention further relates to a computer program product capable of processing values representative of an expression level of the genes AKR1C3 and SPP1 and, optionally, MAP4, CXCL9, PTGER3 and/or VEGFC, by mathematically combining said values to yield a combined score, wherein said combined score is indicative of benefit from cytotoxic chemotherapy comprising microtubule-stabilizing drugs.

Said computer program product may be stored on a data carrier or implemented, on a diagnostic system capable of outputting values representative of expression levels of given genes, such as a real time PCR system.

If the computer program product is stored on a data carrier or running on a computer, operating personal can input the expression values obtained for the expression level of the respective genes. Data can be inputted manually or automatically by a diagnostic system. The computer program product can then apply an algorithm to produce a combined score indicative of benefit from cytotoxic chemotherapy for a given patient.

Preferred algorithms are described below.

Preferably gene expression is determined as an mRNA expression level. Gene expression at the mRNA level may be determined by hybridisation based methods, PCR methods, microarray methods and others. A preferred form is kinetic or quantitative RT-PCR using e.g. commercially available systems such as Tagman, Lightcycler or the like.

It was found that the gene expression of AKR1C3, MAP4, SPP1, PTGER3, VEGFC, and CXCL9 is generally indicative for benefit from taxane-based therapy in patients. For AKR1C3, MAP4, SPP1, PTGER3, and VEGFC a high expression and for CXCL9 a low expression generally indicates an increased likelihood of benefit from taxane-based therapy.

Therefore, in more general terms the disclosure relates to a method or predicting an outcome of cancer, said method comprising:

(a) determining in a biological sample from said patient the expression level of at least one marker gene selected from AKR1C3, MAP4, SPP1, CXCL9, PTGER3, and VEGFC

(b) comparing said expression level to a reference pattern of expression, wherein an increased expression of said at least one marker gene is indicative of said patient having a benefit from microtubule stabilizing agent-based cytotoxic chemotherapy.

Detailed description of the invention

The disclosure is explained in conjunction with exemplary embodiments and the attached figures which show:

Figure 1: ROC curves of an exemplary algorithm (C3) in taxane-treated and not taxane-treated patients for distant metastasis, 5 years. Areas under the curves (AUC) are indicated.

Figure 2: ROC curves of an exemplary algorithm (C3) in taxane-treated and not taxane-treated patients for overall survival, 7 years. Areas under the curves (AUC) are indicated.

Figure 3: ROC curves of an exemplary algorithm (C3c) in taxane-treated and not taxane-treated patients for distant metastasis, 5 years. Areas under the curves (AUC) are indicated.

Figure 4: ROC curves of an exemplary algorithm (C3c) in taxane-treated and not taxane-treated patients for overall survival, 7 years. Areas under the curves (AUC) are indicated.

Figure 5: ROC curves of an exemplary algorithm (C4) in taxane-treated and not taxane-treated patients for distant metastasis, 5 years. Areas under the curves (AUC) are indicated.

Figure 6: ROC curves of an exemplary algorithm (C4) in taxane-treated and not taxane-treated patients for overall survival, 7 years. Areas under the curves (AUC) are indicated.

Figure 7: ROC curves of an exemplary algorithm (C6) in taxane-treated and not taxane-treated patients for distant metastasis, 5 years. Areas under the curves (AUC) are indicated.

Figure 8: ROC curves of an exemplary algorithm (C6) in taxane-treated and not taxane-treated patients for overall survival, 7 years. Areas under the curves (AUC) are indicated.

Figure 9: ROC curves of an exemplary algorithm (C7) in taxane-treated and not taxane-treated patients for distant metastasis, 5 years. Areas under the curves (AUC) are indicated.

Figure 10: ROC curves of an exemplary algorithm (C7) in taxane-treated and not taxane-treated patients for overall survival, 7 years. Areas under the curves (AUC) are indicated.

Figure 11: Graphic representation of the determination of a threshold value for algorithm C3. Dotted lines mark the boundary of the 95% confidence interval.

Figure 12: Graphic representation of the determination of a threshold value for algorithm C3c. Dotted lines mark the boundary of the 95% confidence interval.

Figure 13: Graphic representation of the determination of a threshold value for algorithm C4. Dotted lines mark the boundary of the 95% confidence interval.

Figure 14: Graphic representation of the determination of a threshold value for algorithm C6. Dotted lines mark the boundary of the 95% confidence interval.

Figure 15: Graphic representation of the determination of a threshold value for algorithm C7. Dotted lines mark the boundary of the 95% confidence interval.

Figure 16: Kaplan-Meier survival curves for distant metastasis-free survival (MFS) in all samples (above) and in samples stratified in two groups according C3.

Figure 17: Kaplan-Meier survival curves for distant metastasis-free survival (MFS) in all samples (above) and in samples stratified in two groups according C3c.

Figure 18: Kaplan-Meier survival curves for distant metastasis-free survival (MFS) in all samples (above) and in samples stratified in two groups according C4.

Figure 19: Kaplan-Meier survival curves for distant metastasis-free survival (MFS) in all samples (above) and in samples stratified in two groups according C6.

Figure 20: Kaplan-Meier survival curves for distant metastasis-free survival (MFS) in all samples (above) and in samples stratified in two groups according C7.

Figure 21: Kaplan-Meier survival curves for overall survival in all samples (above) and in samples stratified in two groups according C3.

Figure 22: Kaplan-Meier survival curves for overall survival in all samples (above) and in samples stratified in two groups according C3c.

Figure 23: Kaplan-Meier survival curves for overall survival in all samples (above) and in samples stratified in two groups according C4.

Figure 24: Kaplan-Meier survival curves for overall survival in all samples (above) and in samples stratified in two groups according C6.

Figure 25: Kaplan-Meier survival curves for overall survival in all samples (above) and in samples stratified in two groups according C7.

Herein disclosed is a unique panel of genes which can be combined into an algorithm for the here presented new predictive test.

Table 1: List of Genes used in the methods disclosure herein :

| Gene | Name | Gene ID | Process |
|------|------|---------|---------|
| AKR1C3 | aldo-keto reductase family 1, member C3 | 8644 | metabolism |
| MAP4 | microtubule-associated protein 4 | 4134 | microtubule depolymerization |
| SPP1 | secreted phosphoprotein 1 | 6696 | cell communication |
| VEGFC | vascular endothelial growth factor C | 7424 | angiogenesis |
| PTGER3 | prostaglandin E receptor 3 | 5733 | Signal transduction |
| CXCL9 | chemokine (C-X-C motif) ligand 9 | 4283 | Immune response |
| CALM2 | Calmodulin 2 | 805 | Reference Gene |
| OAZ1 | ornithine decarboxylase antizyme 1 | 4946 | Reference Gene |
| RPL37A | ribosomal protein L37a | 6168 | Reference Gene |

Technically, the method can be practiced using two technologies: 1.) Isolation of total RNA from fresh or fixed tumor tissue and 2.) Kinetic RT-PCR of the isolated nucleic acids.

The assay results can be linked together by a software algorithm computing the likely risk of getting metastasis if treated with taxane or not treated with taxane.

Molecular methods

RNA was isolated from formalin-fixed paraffin-embedded ("FFPE") tumor tissue samples employing an experimental method based on proprietary magnetic beads from Siemens Healthcare Diagnostics. In short, the FFPE slide were lysed and treated with Proteinase K for 2 hours 55°C with shaking. After adding a binding buffer and the magnetic particles (Siemens Healthcare Diagnostics, Cologne, Germany) nucleic acids were bound to the particles within 15 minutes at room temperature. On a magnetic stand the supernatant was taken away and beads were washed several times with washing buffer. After adding elution buffer and incubating for 10 min at 70°C the supernatant was taken away on a magnetic stand without touching the beads. After normal DNAse I treatment for 30 min at 37°C and inactivation of DNAse I the solution was used for reverse transcription-polymerase chain reaction (RT-PCR).

RT-PCR was run as standard kinetic one-step Reverse Transcriptase TaqMan™ polymerase chain reaction (RT-PCR) analysis on a ABI7900 (Applied Biosystems) PCR system for assessment of mRNA expression. Raw data of the RT-PCR can be normalized to one or combinations of the housekeeping genes RPL37A, CALM2, OAZ1 by using the delta CT method, known to those skilled in the art. In brief, a total of 40 cycles of RNA amplification were applied and the cycle threshold (CT) of the target genes was set as being 0.5. CT scores were normalized by subtracting the CT score of the housekeeping gene or the mean of the combinations from the CT score of the target gene (Delta CT). RNA results were then reported as 20-Delta CT, which would correlate to the log2 of the mRNA expression level of the target gene. For each gene specific Primer/Probe were designed by Primer Express ® software v2.0 (Applied Biosystems) according to manufacturers instructions.

In a representative example, quantitative reverse transcriptase PCR was performed according to the following protocol:

Primer/Probe Mix:

| 50 µl | 100 µM | Stock Solution Forward Primer |
|---|---|---|
| 50 µl | 100 µM | Stock Solution Reverse Primer |
| 25 µl | 100 µM | Stock Solution Taq Man Probe |

bring to 1000 µl with water

10 µl Primer/Probe Mix (1:10) are lyophilized, 2.5 h RT

RT-PCR Assay set-up for 1 well:

| 3.1 | µl Water |
|---|---|
| 5.2 | µl RT qPCR MasterMix (Invitrogen) with ROX dye |
| 0.5 | µl MgSO4 (to 5.5 mM final concentration) |
| 1 | µl Primer/Probe Mix dried |
| 0.2 | µl RT/Taq Mx (-RT: 0.08 µL Taq) |
| 1 | µl RNA (1:2) |

Thermal Profile:

RT step

| 50 °C | 30 Min * |
|---|---|
| 8 °C | ca. 20 Min * |
| 95 °C | 2 Min |

PCR cycles (repeated for 40 cycles)

| 95 °C | 15 Sec. |
|---|---|
| 60 °C | 30 Sec. |

Gene expression can be determined by known quantitative PCR methods and devices, such as TaqMan, Lightcycler and the like. It can then be expressed e.g. as cycle threshold value (CT value).

RNA was isolated from 321 formalin-fixed paraffin-embedded (FFPE) samples from node-positive patients with breast cancer having participated in the Greek taxane trial HeCOG 10/97 (chemotherapy regimen: E-T-CMF vs. E-CMF) using a Siemens proprietary isolation technique and expression of the candidate genes was measured with kPCR. Three replicate measurements per gene and sample are taken into account. Quality control is done by estimating the total RNA and DNA amounts and considering measurement noise using a pre-defined noise model. Variations in RNA amount are compensated by subtracting measurement values of reference genes to yield so called delta CT values. Delta CT values are bounded to a gene-independent range to reduce the effect of measurement outliers and to handle PCR fluorescence curves not showing a signal. On the basis of Her2 and ESR1 mRNA expression the samples were separated in three previously described molecular subtypes of breast cancer: Her2-positive (Her2-like), Her2-neg./ESR1-pos. (luminal) and Her2-neg./ESR1-neg. (triple-negative). Her2-positive tumors were excluded from the study. The triple negative tumors were also excluded since the cohort size was too small to get reliable results. For the 208 remaining Her2-negative, ESR1-positive tumors which comprise 60-70% of all breast cancer cases, genes which were able to predict taxane-benefit (differences between treatment arms) were selected by univariate and multivariate COX-regression.

It was found that the gene expression of AKR1C3, MAP4, SPP1, CXCL9, PTGER3, and VEGFC is generally indicative for benefit from taxane-based therapy in patients. For AKR1C3, MAP4, SPP1, PTGER3, and VEGFC a high expression and for CXCL9 a low expression generally indicates an increased likelihood of benefit from taxane-based therapy.

Five exemplary algorithms consisting of three resp. five predictive genes plus three normalization genes were generated and coefficients were estimated by multivariate COX-regression.

Although the genes AKR1C3, MAP4, SPP1, CXCL9, PTGER3, and VEGFC were found to be predictive on samples of patients subjected to taxane-based therapy, obviously, these genes will be also predictive for benefit from other cytotoxic chemotherapy inhibiting cell proliferation by stabilizing microtubules such as for example epothilones.

Table 2: Combination of genes used in exemplary embodiments of the method of the invention:

| Gene | Algorithms | | | | |
|---|---|---|---|---|---|
| | C3 | C3c | C4 | C6 | C7 |
| AKR1C3 | X | X | X | X | X |
| MAP4 | X | X | X | | |
| SPP1 | X | X | X | X | X |
| CXCL9 | | | | X | |
| VEGFC | | | X | | |
| PTGER3 | | | X | | |
| CALM2 | X | X | X | X | X |
| OAZ1 | X | X | X | X | X |
| RPL37A | X | X | X | X | X |
| # genes of interest | 3 | 3 | 5 | 3 | 2 |

Example 1 Algorithm C3

This algorithm is a linear combination of the expression levels of AKR1C3, MAP4, and SPP1. Expression levels are obtained by kinetic PCR and expressed as cycle threshold values relative to reference genes. The algorithm may be described as follows

$$C3 \text{ score}: C3 = 0.235 \ [0.004..0.465] * AKR1C3 + 0.222 \ [0.036..0.407] * MAP4 + 0.293 \ [0.115..0.470] * SPP1$$

The score is a real-valued benefit score reflecting the difference of likelihoods of clinical outcomes in taxane-treated patients and in non-taxane-treated patients. The numbers in squared brackets denote 95% confidence intervals for the coefficients. In other words, instead of multiplying the expression value of AKR1C3 with 0.235, it may be multiplied with any coefficient between 0.004 and 0.465 and still give a score within the 95% confidence interval. The same holds true for the other genes, independently. A high score indicates that the patient is likely to benefit from taxane-based therapy.

Example 2 Algorithm C3c

This algorithm is a linear combination of the expression levels of AKR1C3, MAP4, and SPP1. Expression levels are obtained by kinetic PCR and expressed as cycle threshold values relative to reference genes. The algorithm may be described as follows

```
C3c score: C3c = 0.232 [-0.048..0.511] * AKR1C3 + 0.178
[0.012..0.345] * MAP4 + 0.269 [0.104..0.435] * SPP1
```

The score is a real-valued benefit score reflecting the difference of likelihoods of clinical outcomes in taxane-treated patients and in non-taxane-treated patients. The numbers in squared brackets denote 95% confidence intervals for the coefficients. In other words, instead of multiplying the expression value of AKR1C3 with 0.232, it may be multiplied with any coefficient between -0.048 and 0.511 and still give a score within the 95% confidence interval. The same holds true for the other genes, independently. A high score indicates that the patient is likely to benefit from taxane-based therapy.

Example 3 Algorithm C4

This algorithm is a combination of the expression levels of AKR1C3, MAP4, SPP1, PTGER3, and VEGFC. Expression levels are obtained by kinetic PCR and expressed as cycle threshold values relative to reference genes. The algorithm may be described as follows:

```
C4 score: C4 = 0.214 [0.029..0.399] * MAP4 + 0.305
[0.120..0.490] * SPP1 + 0.538 [0.052..1.024] * motiv1

Whereas motiv1 = 0.295 [0.065..0.525] * AKR1C3 + 0.206
[0.029..0.383] * PTGER3 +
0.385 [0.031..0.738] * VEGFC
```

The score is a real-valued benefit score reflecting the difference of likelihoods of clinical outcomes in taxane-treated patients and in non-taxane-treated patients. The numbers in squared brackets denote 95% confidence intervals for the coefficients. In other words, instead of multiplying the expression value of AKR1C3 with 0.295, it may be multiplied with any coefficient between 0.065 and 0.525 and still give a score within the 95% confidence interval.. The same holds true for the other genes, independently. A high score indicates that the patient is likely to benefit from taxane-based therapy.

Example 4 Algorithm C6

This algorithm is a linear combination of the expression levels of AKR1C3, CXCL9, and SPP1. Expression levels are obtained by kinetic PCR and expressed as cycle threshold values relative to reference genes. The algorithm may be described as follows

```
C6 score: C6 = 0.299 [0.014..0.583] * AKR1C3 + (- 0.163) [-
0.304..-0.022] * CXCL9 + 0.191 [0.043..0.339] * SPP1
```

The score is a real-valued benefit score reflecting the difference of likelihoods of clinical outcomes in taxane-treated patients and in non-taxane-treated patients. The numbers in squared brackets denote 95% confidence intervals for the coefficients. In other words, instead of multiplying the expression value of AKR1C3 with 0.299, it may be multiplied with any coefficient between 0.014 and 0.583 and still give a score within the 95% confidence interval. The same holds true for the other genes, independently. A high score indicates that the patient is likely to benefit from taxane-based therapy.

Example 5 Algorithm C7

This algorithm is a linear combination of the expression levels of AKR1C3 and SPP1. Expression levels are obtained by kinetic PCR and expressed as cycle threshold values relative to reference genes. The algorithm may be described as follows

$$C7\ score:\ C7 = 0.320\ [0.036..0.604] * AKR1C3 + 0.218\ [0.067..0.369] * SPP1$$

The score is a real-valued benefit score reflecting the difference of likelihoods of clinical outcomes in taxane-treated patients and in non-taxane-treated patients. The numbers in squared brackets denote 95% confidence intervals for the coefficients. In other words, instead of multiplying the expression value of AKR1C3 with 0.320, it may be multiplied with any coefficient between 0.036 and 0.604 and still give a score within the 95% confidence interval. The same holds true for the other genes, independently. A high score indicates that the patient is likely to benefit from taxane-based therapy.

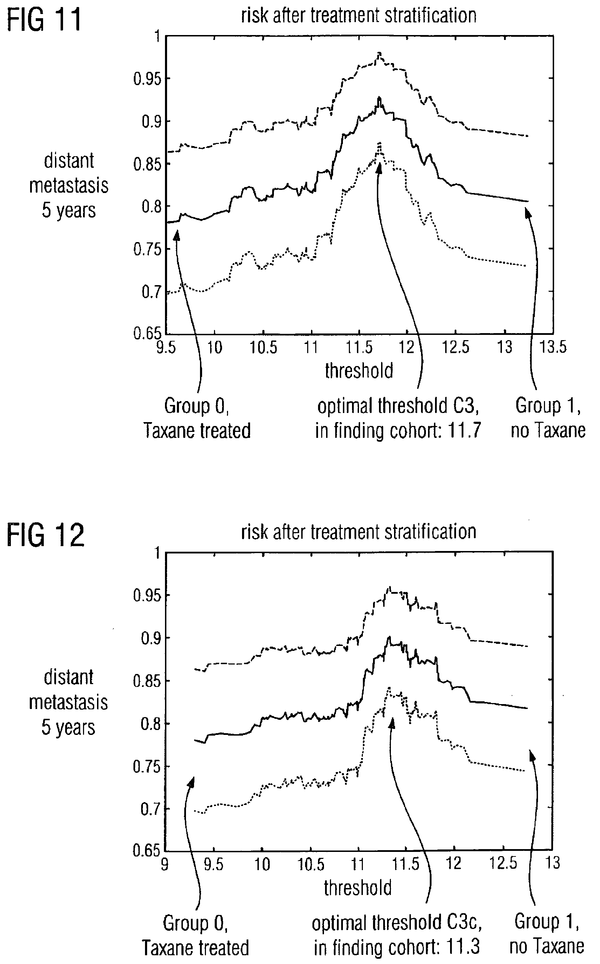

The scores can further be discriminated into two, three or more classes indicating patients to have adverse effect, no additional effect or a benefit from the addition of taxanes to standard chemotherapy. An optimal threshold to discriminate two classes may e.g. be obtained as described below and shown in figs. 11-15 for C3, C3c, C4, C6, and C7:

- stratify patients using the combined score of a given algorithm.
- Determine range of scores (e.g.9.5 to 13.5. for algorithm C3).
- For each possible threshold value (x-axis) within the range, estimate the probability (y-axis) of not having a distant metastasis within 5 years if all patients were treated according to C3's prediction (see below).
- Determine optimal threshold by maximizing said probability. Therefore robustness (small changes on the x-axis due to measurement noise) should be considered.

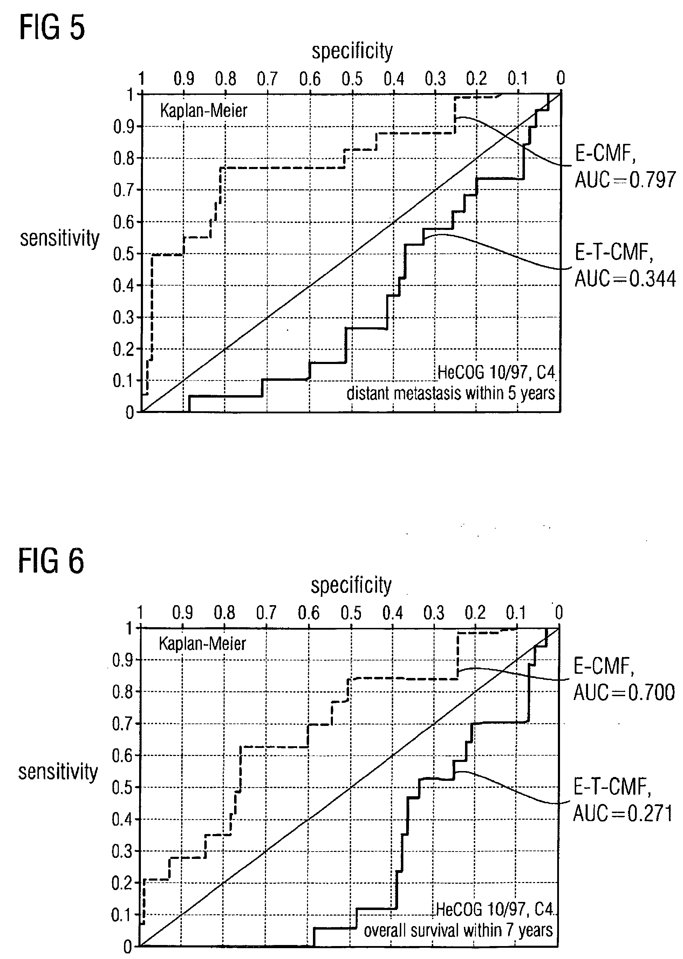

The probability of not developing a distant metastasis within 5 years is estimated on the training data as follows. For a fixed threshold (x-axis) patients are classified as having either predicted "taxane benefit" or "taxane adverse effect" Based on this two sets of samples are selected: Samples predicted "taxane benefit" and treated with E-T-CMF and samples predicted "taxane adverse effect" and treated with E-CMF. The union of theses two sets describes those patients treated according to the C3 prediction, thus the probability of not developing a distant metastasis within 5 years is estimated from it by Kaplan-Meier estimation. In the example shown in figure 5, it is shown that a threshold of 11.7 is optimal. In other words, if patients having C3 scores of > 11.7 are treated with E-T-CMF and patients having C3 scores <= 11.7 are treated with E-CMF, then the probability of not developing a distant metastasis within 5 years is comparatively large (ca. 93%), in particular much larger than if patients were not stratified (ca. 78%) or based on a very different threshold.

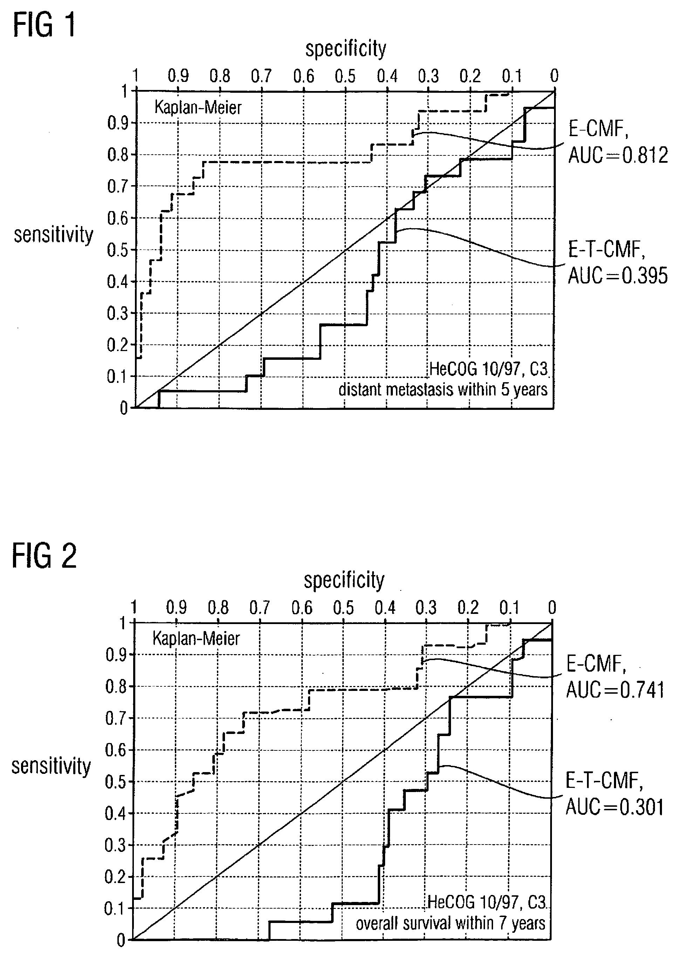

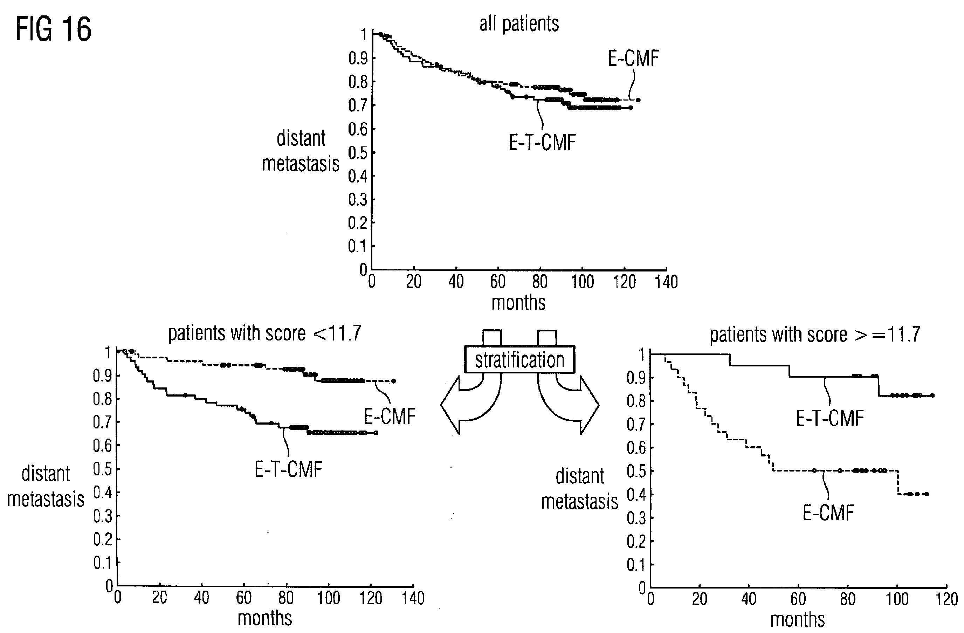

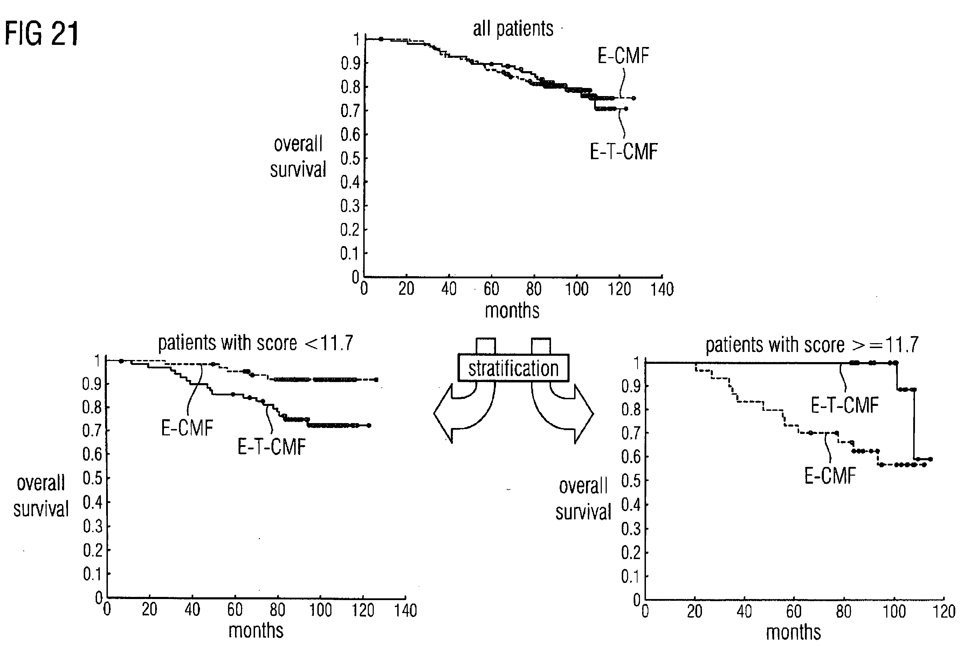

Performance of the five algorithms C3, C3c, C4, C6 and C7 was examined in a cohort of 208 tumor samples of the randomized clinical study HeCOG 10-97. The patients were either treated with epirubicin-doxetaxel-cyclophosphamide-methotrexate-5-fluoruracil (E-T-CMF) adjuvant chemotherapy or with epirubicin-cyclophosphamide-methotrexate-5-fluoruracil (E-CMF) adjuvant chemotherapy. Tumors were classified as "luminal" on the basis of ESR1 and ERBB2 mRNA expression with pre-defined cutoff values (ERBB2 negative and ESR1 positive). C3, C3c, C4, C6, and C7 scores were calculated Results were analysed for the endpoints distant metastasis within 5 years and death within 7 years. Receiver operator characteristics (ROC) of the two predictive scores in the finding cohort showed that they were both positively associated with distant metastasis-free survival and overall survival in the patient group which had not received taxane (Fig. 1-10). In the group with taxane a trend to a negative association with outcome was observed. Remaining risk of patients after stratification by C3, C3c, C4, C6 or C7 and treatment according to this stratification was calculated and plotted as a function of the threshold separating the prediction classes "taxane benefit" and "taxane adverse effect" (Fig. 11-15). On the basis of these plots threshold values were defined for separation of a "taxane benefit" group (>=11.7 for C3; >=11.3 for C3c; >=15.5 for C4; >=6.1 for C6; >=9.4 for C7) and a "taxane adverse effect" group (<11.7 for C3; <11.3 for C3c; <15.5 for C4; <6.1 for C6; <9.4 for C7) by minimizing the remaining risk. Patients were separated into the "taxane benefit" group or the "taxane adverse effect" group using the optimized threshold values of the two scores. As estimated by the Kaplan-Meier method we found in this training cohort that patients with high Taxane Benefit Scores had a benefit from taxane therapy whereas patients with low scores showed a better distant metastasis-free survival if not treated with taxane (Fig. 16-20). This was also true for overall survival (Fig. 21-25).

Results are shown in the figures:

Figure 1: ROC curves of the C3 score in E-T-CMF- (group 0) or E-CMF- (group 1) treated patients for distant metastasis, 5 years. Areas under the curves (AUC) are indicated.

Figure 2: ROC curves of the C3 score in E-T-CMF- (group 0) or E-CMF- (group 1) treated patients for overall survival, 7 years. Areas under the curves (AUC) are indicated.

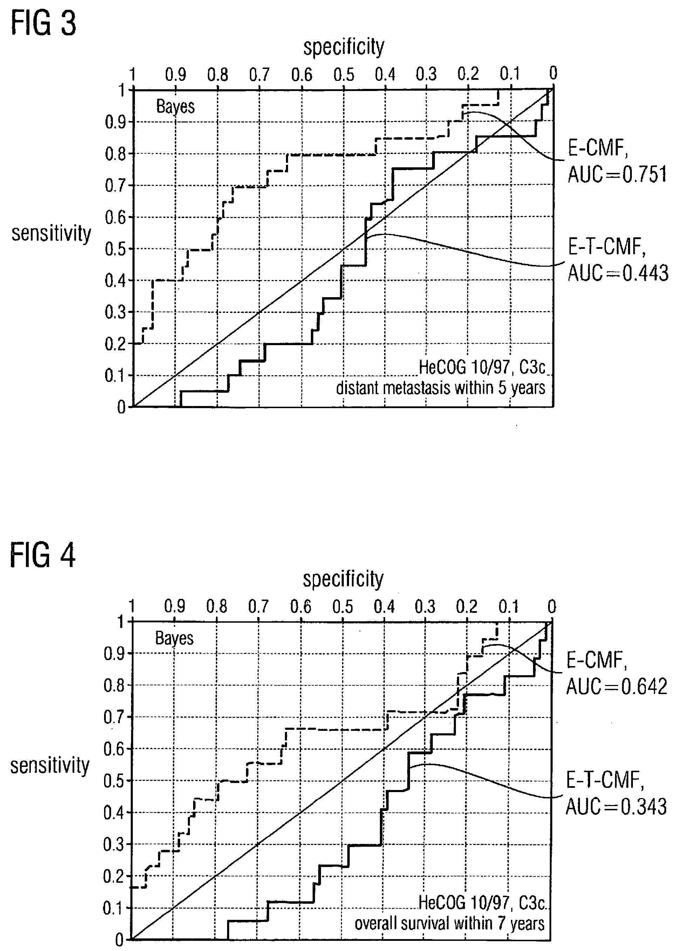

Figure 3: ROC curves of the C3c score in E-T-CMF- (group 0) or E-CMF- (group 1) treated patients for distant metastasis, 5 years. Areas under the curves (AUC) are indicated.

Figure 4: ROC curves of the C3c score in E-T-CMF- (group 0) or E-CMF- (group 1) treated patients for overall survival, 7 years. Areas under the curves (AUC) are indicated.

Figure 5: ROC curves of the C4 score in E-T-CMF- (group 0) or E-CMF- (group 1) treated patients for distant metastasis, 5 years. Areas under the curves (AUC) are indicated.

Figure 6: ROC curves of the C4 score in E-T-CMF- (group 0) or E-CMF- (group 1) treated patients for overall survival, 7 years. Areas under the curves (AUC) are indicated.

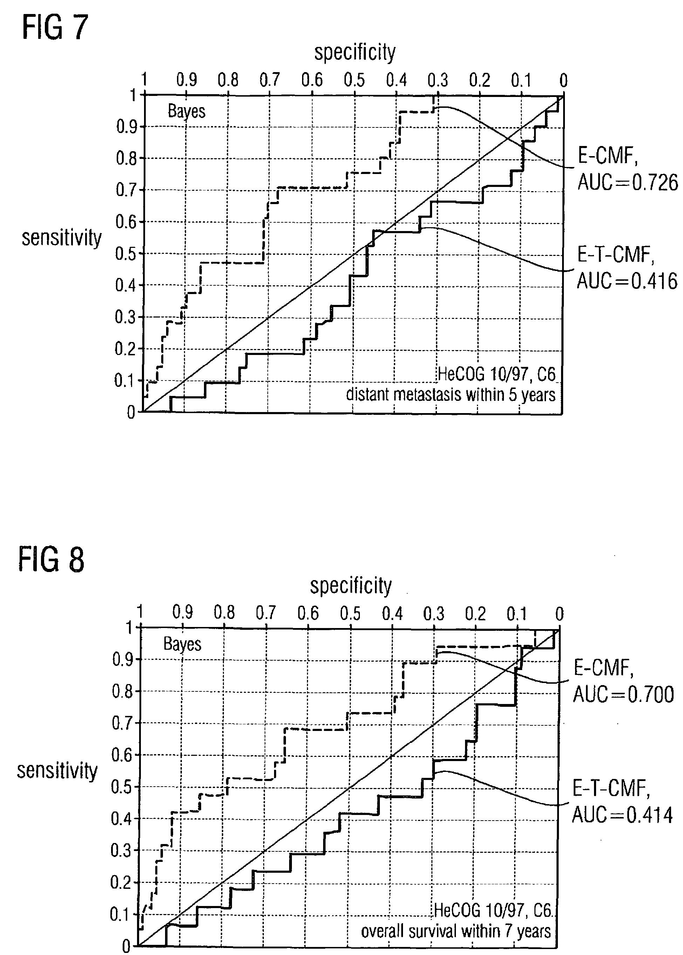

Figure 7: ROC curves of the C6 score in E-T-CMF- (group 0) or E-CMF- (group 1) treated patients for distant metastasis, 5 years. Areas under the curves (AUC) are indicated.

Figure 8: ROC curves of the C6 score in E-T-CMF- (group 0) or E-CMF- (group 1) treated patients for overall survival, 7 years. Areas under the curves (AUC) are indicated.

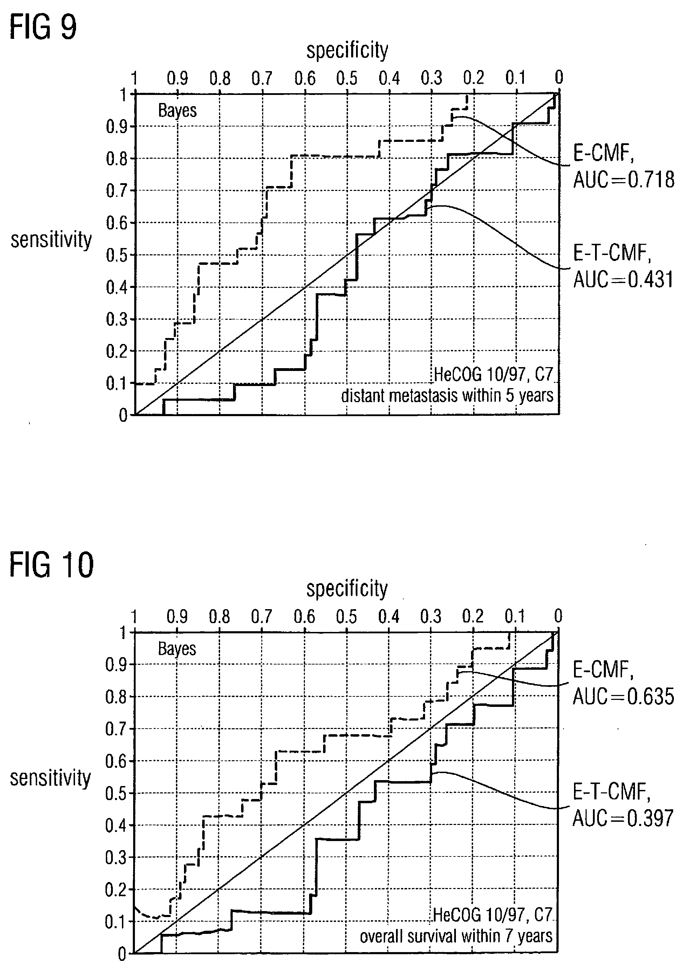

Figure 9: ROC curves of the C7 score in E-T-CMF- (group 0) or E-CMF- (group 1) treated patients for distant metastasis, 5 years. Areas under the curves (AUC) are indicated.

Figure 10: ROC curves of the C7 score in E-T-CMF- (group 0) or E-CMF- (group 1) treated patients for overall survival, 7 years. Areas under the curves (AUC) are indicated.

Risk scores were calculated and patients were discriminated into high, intermediate and low risk by applying two thresholds on the score. The thresholds were chosen by discretizing all samples in quartiles. The low risk group comprises the samples of the first and second quartile, the intermediate and high risk groups consist of the third and fourth quartiles of samples, respectively. Log rank test and log rank test for trend were performed and p values were calculated.

Figure 11: Probability of not developing distant metastasis within 5 years after stratification using C3 benefit scores as a function of the threshold separating prediction classes "taxane benefit" and "taxane adverse effect".The X-axis represents possible thresholds discriminating predicted Taxane-benefit from Taxane-adverse-effect; it covers the whole range of C3 scores within the training cohort. The Y-axis represents the estimated probability of not having a distant metastasis within five years including a 95% confidence interval if patients were treated according to C3's predictions; it thus is a function of the said threshold. The probability on the Y-axis is estimated by a Kaplan-Meier statistic based on those patients treated as predicted, i.e. patients treated with Taxane (E-T-CMF) and having a C3 score above the threshold (predicted "taxane benefit") plus patients not treated with Taxane (E-CMF) and having a C3 score below the threshold (predicted "taxane adverse effect"). Dotted lines mark the boundary of the 95% confidence interval.

Figure 12: Probability of not developing distant metastasis within 5 years after stratification using C3c benefit scores as a function of the threshold separating prediction classes "taxane benefit" and "taxane adverse effect".(details see figure legend 11). Dotted lines mark the boundary of the 95% confidence interval.

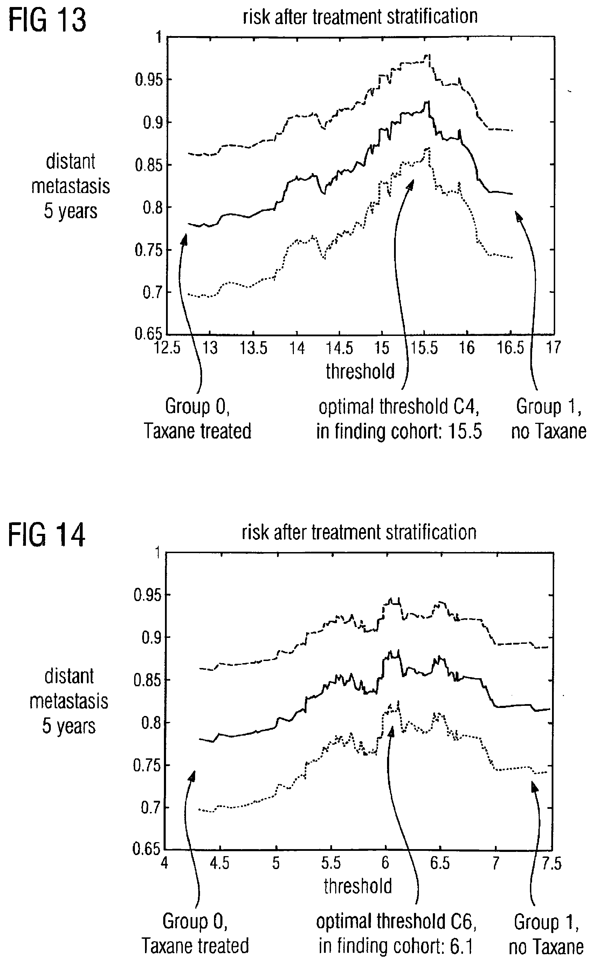

Figure 13: Probability of not developing distant metastasis within 5 years after stratification using C4 benefit scores as a function of the threshold separating prediction classes "taxane benefit" and "taxane adverse effect".(details see figure legend 11). Dotted lines mark the boundary of the 95% confidence interval.

Figure 14: Probability of not developing distant metastasis within 5 years after stratification using C6 benefit scores as a function of the threshold separating prediction classes "taxane benefit" and "taxane adverse effect".(details see figure legend 11). Dotted lines mark the boundary of the 95% confidence interval.

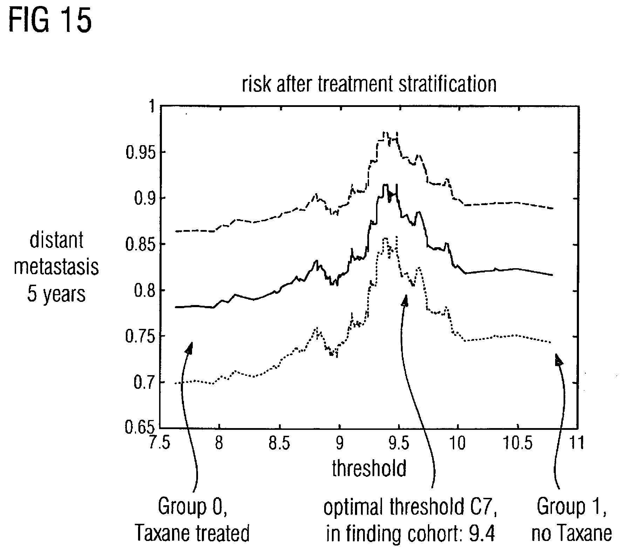

Figure 15: Probability of not developing distant metastasis within 5 years after stratification using C7 benefit scores as a function of the threshold.separating prediction classes "taxane benefit" and "taxane adverse effect".(details see figure legend 11). Dotted lines mark the boundary of the 95% confidence interval.

Figure 16: Kaplan-Meier curves of the estimated probability of not getting a distant metastasis in all samples (above) and in samples stratified in two groups according C3 (below left: "taxane adverse effect" group; below right: "taxane benefit" group). Log rank test was performed and P-values and hazard ratios were calculated.
C3, distant metastasis, patients with risk <11.7: n = 139

```
P(LogRankTest) = 0.001
```

```
hazard ratio = 0.266 (0.114 - 0.621)
```

```
P(hazard ratio) = 0.002
```

C3, distant metastasis, patients with risk >=11.7: n = 51

```
P(LogRankTest) = 0.003
```

```
hazard ratio = 5.349 (1.553 - 18.428)
```

```
P(hazard ratio) = 0.008
```

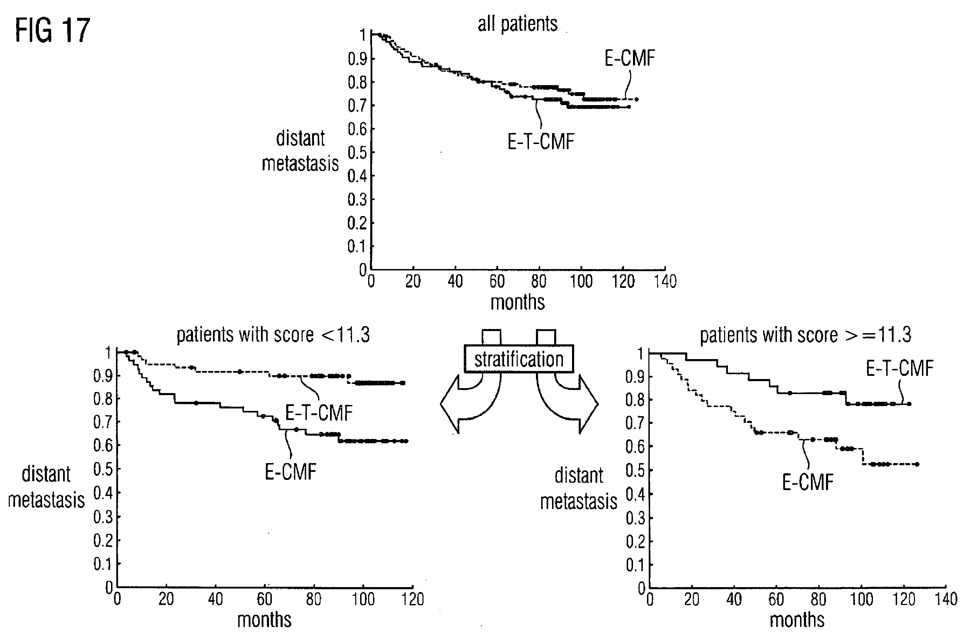

Figure 17: Kaplan-Meier curves of the estimated probability of not getting a distant metastasis in all samples (above) and in samples stratified in two groups according C3c (below left: "taxane adverse effect" group; below right: "taxane benefit" group). Log rank test was performed and P-values and hazard ratios were calculated. C3c, distant metastasis, patients with risk <11.3: n = 117

```
P(LogRankTest) = 0.002
```

```
hazard ratio = 0.274 (0.116 - 0.648)
```

```
P(hazard ratio) = 0.003
```

C3c, distant metastasis, patients with risk >=11.3: n = 79

```
P(LogRankTest) = 0.029
```

```
hazard ratio = 2.563 (1.068 - 6.149)
```

```
P(hazard ratio) = 0.035
```

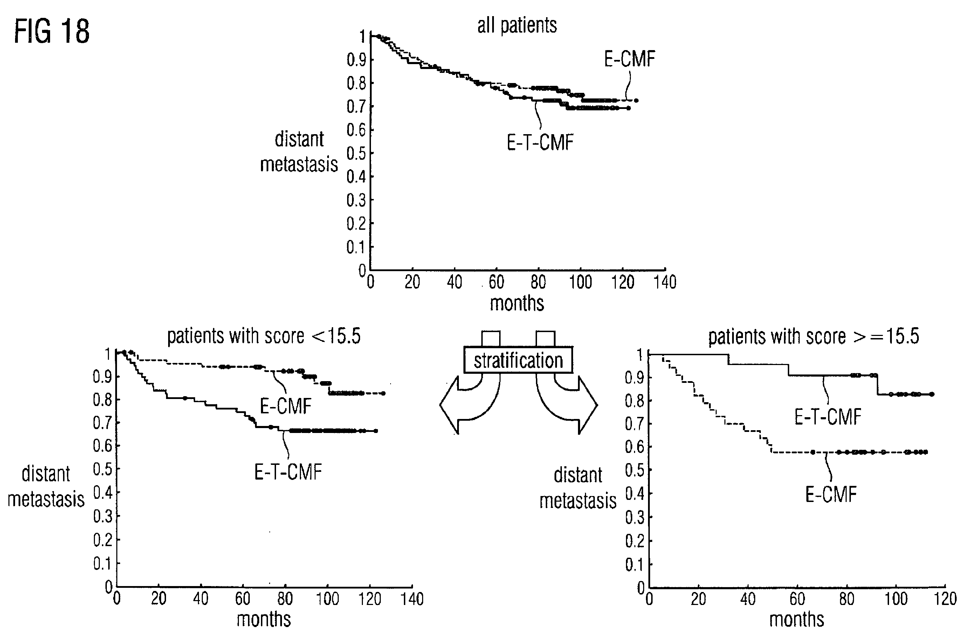

Figure 18: Kaplan-Meier curves the estimated the probability of not getting a distant metastasis in all samples (above) and in samples stratified in two groups according C4 (below left: "taxane adverse effect" group; below right: "taxane benefit" group). Log rank test was performed and P-values and hazard ratios were calculated.
C4, distant metastasis, patients with risk <15.5: n = 131

```
P(LogRankTest) = 0.004
```

```
hazard ratio = 0.327 (0.146 - 0.735)
```

$$P(hazard\ ratio) = 0.007$$

C4, distant metastasis, patients with risk >=15.5: n = 55

$$P(LogRankTest) = 0.016$$

$$hazard\ ratio = 4.133\ (1.184 - 14.428)$$

$$P(hazard\ ratio) = 0.026$$

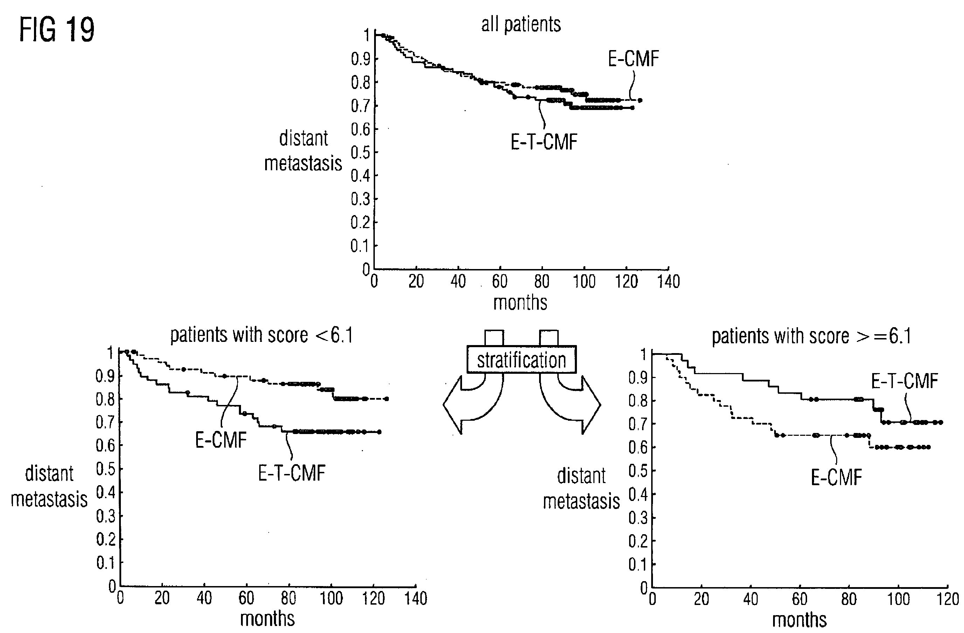

Figure 19: Kaplan-Meier curves the estimated the probability of not getting a distant metastasis in all samples (above) and in samples stratified in two groups according C6 (below left: "taxane adverse effect" group; below right: "taxane benefit" group). Log rank test was performed and P-values and hazard ratios were calculated.
C6, distant metastasis, patients with risk <6.1: n = 126

$$P(LogRankTest) = 0.022$$

$$hazard\ ratio = 0.430\ (0.204 - 0.903)$$

$$P(hazard\ ratio) = 0.026$$

C6, distant metastasis, patients with risk >=6.1: n = 76

$$P(LogRankTest) = 0.154$$

$$hazard\ ratio = 1.811\ (0.791 - 4.146)$$

$$P(hazard\ ratio) = 0.160$$

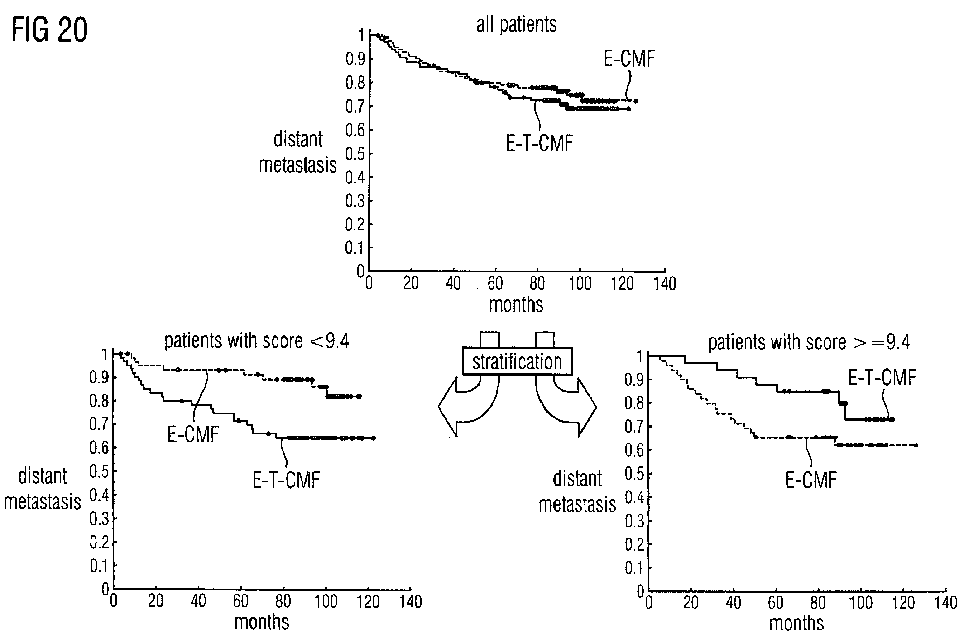

Figure 20: Kaplan-Meier curves the estimated the probability of not getting a distant metastasis in all samples (above) and in samples stratified in two groups according C7 (below left: "taxane adverse effect" group; below right: "taxane benefit" group). Log rank test was performed and P-values and hazard ratios were calculated.
C7, distant metastasis, patients with risk <9.4: n = 120

$$P(LogRankTest) = 0.006$$

$$hazard\ ratio = 0.338\ (0.149 - 0.763)$$

$$P(hazard\ ratio) = 0.009$$

C7, distant metastasis, patients with risk >=9.4: n = 82

$$P(LogRankTest) = 0.094$$

$$hazard\ ratio = 2.076\ (0.866 - 4.974)$$

$$P(hazard\ ratio) = 0.102$$

Figure 21: Kaplan-Meier curves of the estimated probability of death in all samples (above) and in samples stratified in two groups according C3 (below left: "taxane adverse effect" group; below right: "taxane benefit" group). Log rank test was performed and P-values and hazard ratios were calculated.
C3, overall survival, patients with risk <11.7: n = 139

$$P(LogRankTest) = 0.005$$

$$hazard\ ratio = 0.265\ (0.098 - 0.713)$$

$$P(hazard\ ratio) = 0.009$$

C3, overall survival, patients with risk >=11.7: n = 51

$$P(LogRankTest) = 0.007$$

$$hazard\ ratio = 6.288\ (1.380 - 28.641)$$

$$P(hazard\ ratio) = 0.017$$

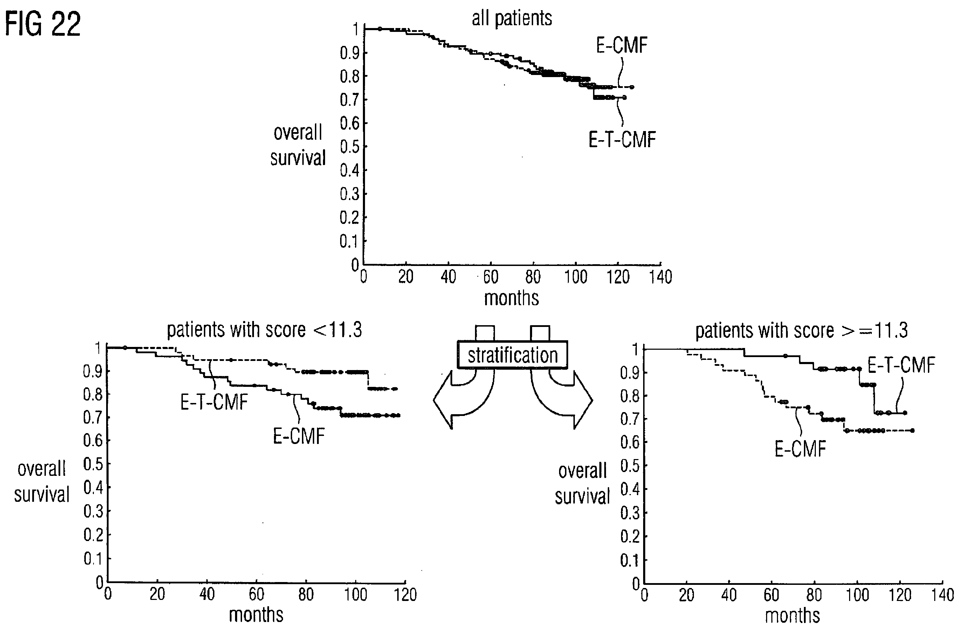

Figure 22: Kaplan-Meier curves of the estimated probability of death in all samples (above) and in samples stratified in two groups according C3c (below left: "taxane adverse effect" group; below right: "taxane benefit" group). Log rank test was performed and P-values and hazard ratios were calculated.
C3c, overall survival, patients with risk <11.3: n = 117

$$P(LogRankTest) = 0.036$$

$$hazard\ ratio = 0.396\ (0.161 - 0.971)$$

$$P(hazard\ ratio) = 0.043$$

C3c, overall survival, patients with risk >=11.3: n = 79

$$P(LogRankTest) = P(LogRankTest) = 0.038$$

```
hazard ratio = 2.825 (1.013 - 7.877)
```

```
P(hazard ratio) = 0.047
```

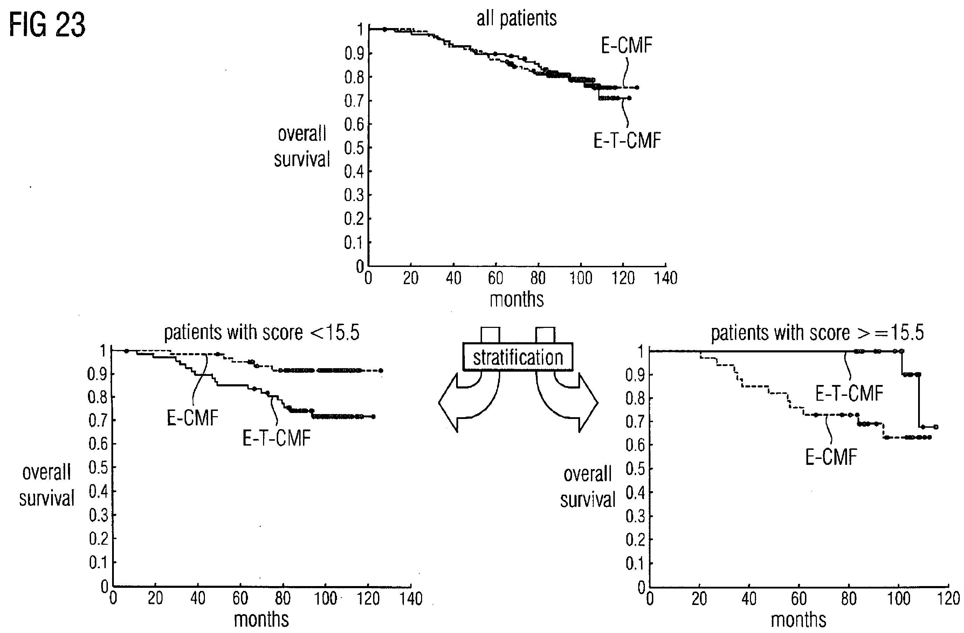

Figure 23: Kaplan-Meier curves of the estimated probability of death in all samples (above) and in samples stratified in two groups according C4 (below left: "taxane adverse effect" group; below right: "taxane benefit" group). Log rank test was performed and P-values and hazard ratios were calculated.

C4, overall survival, patients with risk <15.5: n = 131

```
P(LogRankTest) = 0.006
```

```
hazard ratio = 0.274 (0.102 - 0.738)
```

```
P(hazard ratio) = 0.010
```

C4, overall survival, patients with risk >=15.5: n = 55

```
P(LogRankTest) = 0.021
```

```
hazard ratio = 5.025 (1.101 - 22.928)
```

```
P(hazard ratio) = 0.037
```

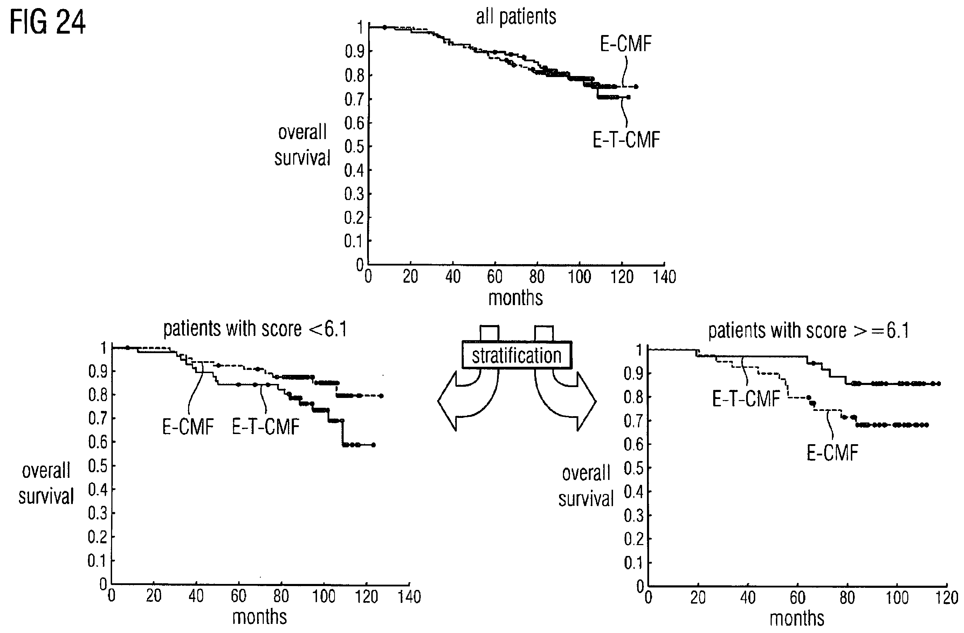

Figure 24: Kaplan-Meier curves of the estimated probability of death in all samples (above) and in samples stratified in two groups according C6 (below left: "taxane adverse effect" group; below right: "taxane benefit" group). Log rank test was performed and P-values and hazard ratios were calculated.

C6, overall survival, patients with risk <6.1: n = 126

```
P(LogRankTest) = 0.084
```

```
hazard ratio = 0.505 (0.229 - 1.113)
```

```
P(hazard ratio) = 0.090
```

C6, overall survival, patients with risk >=6.1: n = 76

```
P(LogRankTest) = 0.069
```

```
hazard ratio = 2.549 (0.896 - 7.248)
```

```
P(hazard ratio) = 0.079
```

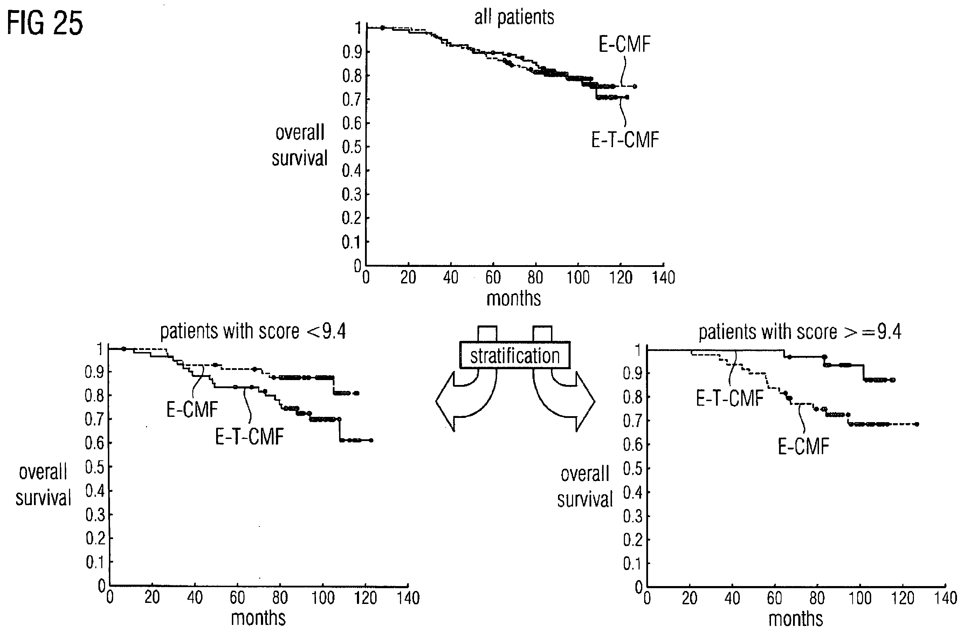

Figure 25: Kaplan-Meier curves of the estimated probability of death in all samples (above) and in samples stratified in two groups according C7 (below left: "taxane adverse effect" group; below right: "taxane benefit" group). Log rank test was performed and P-values and hazard ratios were calculated.

C7, overall survival, patients with risk <9.4: n = 120

$$P(LogRankTest) = 0.045$$

$$hazard\ ratio = 0.437\ (0.190 - 1.006)$$

$$P(hazard\ ratio) = 0.052$$

C7, overall survival, patients with risk >=9.4: n = 82

$$P(LogRankTest) = 0.024$$

$$hazard\ ratio = 3.804\ (1.090 - 13.274)$$

$$P(hazard\ ratio) = 0.036$$

Example 3 composite score

In another exemplary embodiment, the expression level of each gene is determined relative to a reference value. This reference value may be derived from a reference group of patients or healthy subjects. If the expression level of a given gene is above the reference value, this gene is assigned an individual score of +1 and -1 if it is below. The composite score is the sum of the individual scores and a composite score >0 indicates high likelihood of benefit from taxane based therapy. Instead of +1 and -1, different values may be used for weighting the respective genes.

Table 3 shows the sequences of the respective primers and probes used in the examples above.

Table 3

| Seq Id | Gene symbol | Primer-ID | Probe |
|---|---|---|---|
| 1 | MAP4 | SC083 | CGGGGCAGTTGCAGTGGTG |
| 2 | AKR1 C3 | BC468 | AGTCAACTGGAACTCAAAAACCTGCACGTT |
| 3 | PTGER3 | CAGMC315 | TCGGTCTGCTGGTCTCCGCTCC |
| 4 | SPP1 | SC404-MGB | CGACCAAGGAAAACT |
| 5 | VEGFC | R14 | TTGAGTCATCTCCAGCATCCGAGGAAA |
| 6 | AKR1C3 | BC550-MGB | CTGTTTAGCGACTTCAG |
| 7 | MAP4 | BC548-MGB | TGGTCATGGAGTAGAAGG |
| 8 | MAP4 | R187 | AGAAGCGGGCCTCACCATCCAAG |
| 9 | CXCL9 | CAGMC154 | CCACTAACCGACTTGGCTGCTTCCTCTAG |
|  |  |  |  |
| Seq Id | Gene symbol | Primer-ID | Forward Primer |
| 10 | MAP4 | SC083 | CCTACCTCCTCACGGCTCTTC |
| 11 | AKR1C3 | BC468 | CTACAATGAGCAGCGCATCAG |
| 12 | PTGER3 | CAGMC315 | CTGATTGAAGATCATTTTCAACATCA |

(continued)

| Seq Id | Gene symbol | Primer-ID | Forward Primer |
|---|---|---|---|
| 13 | **SPP1** | SC404-MGB | CAGCCTTCTCAGCCAAACG |
| 14 | **VEGFC** | R14 | CCACAGATGTCATGGAATCCAT |
| 15 | **AKR1C3** | BC550-MGB | GACTGGACATATCACCTCTACTTAAATCC |
| 16 | **MAP4** | BC548-MGB | GATTGAAGATACTCCATCTTCTAAACCA |
| 17 | **MAP4** | R187 | GGCCTTCCATCTTACCTTCAAAA |
| 18 | **CXCL9** | CAGMC154 | AAAGGGAA CGGTGAAGTACTAAGC |
|  |  |  |  |
| Seq Id | Gene symbol | Primer-ID | Reverse Primer |
| 19 | **MAP4** | SC083 | AATGTCTGGAGATGGTTCTGTTAATG |
| 20 | **AKR1C3** | BC468 | GGAGATTTCTGTCTAGGCCATCTATG |
| 21 | **PTGER3** | CAGMC315 | GACGGCCATTCAGCTTATGG |
| 22 | **SPP1** | SC404-MGB | CAAATCACTGCAATTCTCATGGTAGT |
| 23 | **VEGFC** | R14 | TGCCTGGCTCAGGAAGATTT |
| 24 | **AKR1C3** | BC550-MGB | TGTCTTTCTGGCCTATGGACTCA |
| 25 | **MAP4** | BC548-MGB | TTCAGTTGGAGACCCTGTAGTATCG |
| 26 | **MAP4** | R187 | TTTTGCAACAGTCTGAGTGCTCTT |
| 27 | **CXCL9** | CAGMC154 | AACTGGGCACCAATCATGCT |

**Claims**

1. Method for predicting an outcome of cancer in a patient, said method comprising:

    (a) determining in a biological sample from said patient the expression levels of the genes AKR1C3, and SPP1,
    (b) mathematically combining said expression levels to yield a combined score, wherein said combined score is indicative of benefit from microtubule stabilizing agent-based cytotoxic chemotherapy of said patient,

wherein an increased level of gene expression of AKR1C3 and SPP1 in comparison to a reference pattern of expression is indicative of a benefit from microtubule stabilizing agent-based cytotoxic chemotherapy.

2. Method of claim 1 wherein further the expression levels of the genes PTGER3, MAP4, CXCL9, and/or VEGFC are determined.

3. Method of claim 1 or 2, wherein one, two or more thresholds are determined for said combined score and discriminated into (1) "predicted benefit" and "predicted non-benefit", (2) "predicted benefit" and "predicted adverse effect", (3) "predicted benefit", "predicted indifferent effect" and "predicted adverse effect", or more risk groups with different probabilities of benefit by applying the threshold on the combined score.

4. Method of any one of the preceding claims wherein said prediction of outcome is the determination of the risk of recurrence of cancer in said patient within 5 to 10 years or the risk of developing distant metastasis in a similar time horizon, or the prediction of death or of death after recurrence.

5. Method of any one of the preceding claims, wherein said determination of expression levels is in a formalin-fixed paraffin embedded sample, in a fixed sample, in a fresh sample or in a fresh-frozen sample.

6. Method of any one of the preceding claims, wherein the patient has previously received treatment by surgery and cytotoxic chemotherapy.

7. Method of any one of the preceding claims, wherein the microtubule stabilizing agent-based cytotoxic chemotherapy is a taxane-based cytotoxic chemotherapy.

8. Method of any one of the preceding claims, wherein said step of mathematically combining comprises a step of applying an algorithm to values representative of expression levels of given genes.

9. Method of claim 8, wherein said algorithm is a linear combination of said values representative of expression levels of given genes.

10. Method of claim 8 or 9 wherein a value for a representative of an expression level of a given gene is multiplied with a coefficient.

11. Method of any one of the preceding claims wherein the cancer is breast cancer.

12. Method of any one of the preceding claims wherein the expression levels are determined as mRNA expression levels by at least one of a hybridization based method, a PCR based method, an array based method.

13. Method of any one of the preceding claims wherein the sample is one of a FFPE tissue sample, a fresh frozen tissue sample, a needle biopsy.

14. Use of a kit for performing a method of any of claims 1 to 13, said kit comprising a set of oligonucleotides capable of specifically binding sequences of the genes AKR1C3 and SPP1 and, optionally, MAP4, CXCL9, PTGER3 and/or VEGFC, or fragments thereof.

15. A computer program product adapted to process values representative of an expression level of the genes AKR1C3 and SPP1 and, optionally, MAP4, CXCL9, PTGER3 and/or VEGFC, by mathematically combining said values to yield a combined score, wherein said combined score is indicative of benefit from microtubule stabilizing agent-based cytotoxic chemotherapy of said patient.

**Patentansprüche**

1. Verfahren zur Voraussage eines Ergebnisses von Krebs in einem Patienten, wobei das Verfahren umfasst:

(a) Bestimmen der Expressionsniveaus der Gene AKR1C3 und SPP1 in einer biologischen Probe von dem Patienten,
(b) mathematisches Kombinieren der Expressionsniveaus, um einen kombinierten Wert zu ergeben, wobei der kombinierte Wert einen Nutzen des Patienten von Mikrotubuli-Stabilisator-basierter zytotoxischer Chemotherapie anzeigt,

wobei ein erhöhtes Niveau an Genexpression von AKR1C3 und SPP1 im Vergleich zu einem Referenzexpressionsmuster einen Nutzen von Mikrotubuli-Stabilisator-basierter zytotoxischer Chemotherapie anzeigt.

2.  Verfahren nach Anspruch 1, wobei ferner die Expressionsniveaus der Gene PTGER3, MAP4, CXCL9 und/oder VEGFC bestimmt werden.

3.  Verfahren nach Anspruch 1 oder 2, wobei einer, zwei oder mehr Schwellenwerte für den kombinierten Wert bestimmt werden und in (1) "vorausgesagter Nutzen" und "vorausgesagter Nicht-Nutzen", (2) "vorausgesagter Nutzen" und "vorausgesagte negative Wirkung", (3) "vorausgesagter Nutzen", "vorausgesagte indifferente Wirkung" und "vorausgesagte negative Wirkung" oder mehr Risikogruppen mit unterschiedlichen Wahrscheinlichkeiten eines Nutzens durch Anwenden des Schwellenwerts auf den kombinierten Wert unterschieden werden.

4.  Verfahren nach einem der vorhergehenden Ansprüche, wobei die Voraussage eines Ergebnisses die Bestimmung des Risikos eines Wiederauftretens von Krebs in dem Patienten innerhalb von 5 bis 10 Jahren oder des Risikos einer Entwicklung von Fernmetastasen in einem ähnlichen Zeithorizont oder die Voraussage von Tod oder von Tod nach Wiederauftreten ist.

5.  Verfahren nach einem der vorhergehenden Ansprüche, wobei die Bestimmung von Expressionsniveaus in einer Formalin-fixierten, Paraffin-eingebetteten Probe, in einer fixierten Probe, in einer frischen Probe oder in einer frisch eingefrorenen Probe erfolgt.

6.  Verfahren nach einem der vorhergehenden Ansprüche, wobei der Patient zuvor Behandlung durch Chirurgie und zytotoxische Chemotherapie erhalten hat.

7.  Verfahren nach einem der vorhergehenden Ansprüche, wobei die Mikrotubuli-Stabilisator-basierte zytotoxische Chemotherapie eine Taxan-basierte zytotoxische Chemotherapie ist.

8.  Verfahren nach einem der vorhergehenden Ansprüche, wobei der Schritt eines mathematischen Kombinierens einen Schritt eines Anwendens eines Algorithmus auf Werte, die für Expressionsniveaus von bestimmten Genen repräsentativ sind, umfasst.

9.  Verfahren nach Anspruch 8, wobei der Algorithmus eine lineare Kombination der Werte, die für Expressionsniveaus von bestimmten Genen repräsentativ sind, ist.

10. Verfahren nach Anspruch 8 oder 9, wobei ein Wert für einen Vertreter eines Expressionsniveaus eines bestimmten Gens mit einem Koeffizienten multipliziert wird.

11. Verfahren nach einem der vorhergehenden Ansprüche, wobei der Krebs Brustkrebs ist.

12. Verfahren nach einem der vorhergehenden Ansprüche, wobei die Expressionsniveaus als mRNA-Expressionsniveaus durch mindestens eines aus einem Hybridisierung-basierten Verfahren, einem PCR-basierten Verfahren und einem Array-basierten Verfahren bestimmt werden.

13. Verfahren nach einem der vorhergehenden Ansprüche, wobei die Probe eine aus einer FFPE-Gewebeprobe, einer frisch eingefrorenen Gewebeprobe und einer Nadelbiopsie ist.

14. Verwendung eines Kits zur Durchführung eines Verfahrens nach einem der Ansprüche 1 bis 13, wobei das Kit einen Satz von Oligonukleotiden, die zur spezifischen Bindung von Sequenzen der Gene AKR1C3 und SPP1 und, gegebenenfalls, MAP4, CXCL9, PTGER3 und/oder VEGFC oder von Fragmenten davon fähig sind, umfasst.

15. Computerprogrammprodukt, das zum Prozessieren von Werten, die für ein Expressionsniveau der Gene AKR1C3 und SPP1 und, gegebenenfalls, MAP4, CXCL9, PTGER3 und/oder VEGFC repräsentativ sind, durch mathematisches Kombinieren der Werte, um einen kombinierten Wert zu erhalten, angepasst ist, wobei der kombinierte Wert einen Nutzen des Patienten von Mikrotubuli-Stabilisator-basierter zytotoxischer Chemotherapie anzeigt.

**Revendications**

1.  Procédé pour la prédiction d'une évolution d'un cancer chez un patient, ledit procédé comprenant:

    (a) la détermination dans un échantillon biologique provenant d'un patient des niveaux d'expression des gènes AKR1C3, et SPP1,
    (b) la combinaison mathématique desdits niveaux d'expression pour donner une cotation combinée, dans laquelle ladite cotation combinée est indicatrice du bénéfice tiré d'une chimiothérapie cytotoxique fondée sur un agent de stabilisation des microtubules dudit patient,

    tandis qu'un niveau accru d'expression génique de AKR1C3 et SPP1 par comparaison avec un modèle d'expression de référence est indicatif d'un bénéfice tiré d'une chimiothérapie cytotoxique fondée sur un agent de stabilisation des microtubules.

2.  Procédé selon la revendication 1, dans lequel on détermine en outre les niveaux d'expression des gènes PTGER3, MAP4, CXCL9 et/ou VEGFC.

3.  Procédé selon la revendication 1 ou 2, dans lequel un, deux ou plus de deux seuils sont déterminés pour ladite cotation combinée et répartis en (1) "bénéfice prédit" et "non-bénéfice prédit", (2) "bénéfice prédit" et "effet défavorable prédit", (3) "bénéfice prédit", "effet indifférent prédit" et "effet défavorable prédit", ou plus de groupes de risques avec différentes probabilités de bénéfices par application du seuil sur la cotation combinée.

4.  Procédé selon l'une quelconque des revendications précédentes, dans lequel ladite prédiction d'évolution est la détermination du risque de récurrence de cancer chez ledit patient en 5 à 10 ans ou le risque de développement de métastases distantes à un horizon temporel similaire, ou la prédiction de mort ou de mort après récurrence.

5.  Procédé selon l'une quelconque des revendications précédentes, dans lequel ladite détermination des niveaux d'expression s'effectue dans un échantillon enrobé de paraffine fixé par le formol, dans un échantillon fixé, dans un échantillon frais ou dans un échantillon frais congelé.

6.  Procédé selon l'une quelconque des revendications précédentes, dans lequel le patient a préalablement reçu un traitement par chirurgie et chimiothérapie cytotoxique.

7.  Procédé selon l'une quelconque des revendications précédentes, dans lequel la chimiothérapie cytotoxique fondée sur un agent de stabilisation des microtubules est une chimiothérapie cytotoxique fondée sur le taxane.

8.  Procédé selon l'une quelconque des revendications précédentes, dans lequel ladite étape de combinaison mathématique comprend une étape d'application d'un algorithme à des valeurs représentatives de niveaux d'expression de gènes donnés.

9.  Procédé selon la revendication 8, dans lequel ledit algorithme est une combinaison linéaire desdites valeurs représentatives de niveaux d'expression de gènes donnés.

10. Procédé selon la revendication 8 ou 9, dans lequel une valeur pour un représentant d'un niveau d'expression d'un gène donné est multipliée par un coefficient.

11. Procédé selon l'une quelconque des revendications précédentes, dans lequel le cancer est un cancer du sein.

12. Procédé selon l'une quelconque des revendications précédentes, dans lequel les niveaux d'expression sont déterminés sous la forme de niveaux d'expression d'ARNm par au moins l'une parmi une méthode fondée sur l'hybridation, une méthode fondée sur une ACP, une méthode fondée sur une répartition matricielle.

13. Procédé selon l'une quelconque des revendications précédentes, dans lequel l'échantillon est l'un parmi un échantillon de tissu FFPE, un échantillon de tissu frais congelé, une biopsie à l'aiguille.

14. Utilisation d'un kit pour la mise en oeuvre d'un procédé selon l'une quelconque des revendications 1 à 3, ledit kit comprenant un ensemble d'oligonucléotides capables de se lier spécifiquement à des séquences des gènes AKR1C3 et SPP1 et, en option, MAP4, CXCL9, PTGER3 et/ou VEGFC, ou des fragments de celles-ci.

15. Produit de programme d'ordinateur adapté pour traiter des valeurs représentatives d'un niveau d'expression des gènes AKR1C3 et SPP1 et, en option, MAP4, CXCL9, PTGER3 et/ou VEGFC, par combinaison mathématique desdites valeurs pour obtenir une cotation combinée, tandis que ladite cotation combinée est indicative d'un bénéfice tiré d'une chimiothérapie cytotoxique fondée sur un agent de stabilisation des microtubules dudit patient.

## FIG 1

Kaplan-Meier — specificity / sensitivity ROC plot.
E-CMF, AUC=0.812
E-T-CMF, AUC=0.395
HeCOG 10/97, C3
distant metastasis within 5 years

## FIG 2

Kaplan-Meier — specificity / sensitivity ROC plot.
E-CMF, AUC=0.741
E-T-CMF, AUC=0.301
HeCOG 10/97, C3
overall survival within 7 years

## FIG 3

## FIG 4

FIG 5

FIG 6

## FIG 7

specificity

sensitivity

Bayes

E-CMF,
AUC=0.726

E-T-CMF,
AUC=0.416

HeCOG 10/97, C6
distant metastasis within 5 years

## FIG 8

specificity

sensitivity

Bayes

E-CMF,
AUC=0.700

E-T-CMF,
AUC=0.414

HeCOG 10/97, C6
overall survival within 7 years

## FIG 9

Bayes

specificity / sensitivity ROC curve

E-CMF, AUC=0.718

E-T-CMF, AUC=0.431

HeCOG 10/97, C7
distant metastasis within 5 years

## FIG 10

Bayes

specificity / sensitivity ROC curve

E-CMF, AUC=0.635

E-T-CMF, AUC=0.397

HeCOG 10/97, C7
overall survival within 7 years

## FIG 11

risk after treatment stratification

distant
metastasis
5 years

threshold

Group 0,
Taxane treated

optimal threshold C3,
in finding cohort: 11.7

Group 1,
no Taxane

## FIG 12

risk after treatment stratification

distant
metastasis
5 years

threshold

Group 0,
Taxane treated

optimal threshold C3c,
in finding cohort: 11.3

Group 1,
no Taxane

FIG 13

risk after treatment stratification

distant metastasis 5 years

threshold

Group 0, Taxane treated

optimal threshold C4, in finding cohort: 15.5

Group 1, no Taxane

FIG 14

risk after treatment stratification

distant metastasis 5 years

threshold

Group 0, Taxane treated

optimal threshold C6, in finding cohort: 6.1

Group 1, no Taxane

## FIG 15

risk after treatment stratification

distant metastasis 5 years

threshold

Group 0, Taxane treated

optimal threshold C7, in finding cohort: 9.4

Group 1, no Taxane

FIG 16

FIG 17

FIG 18

## FIG 19

FIG 20

all patients

distant
metastasis

E-CMF

E-T-CMF

stratification

patients with score <9.4

distant
metastasis

E-CMF

E-T-CMF

months

patients with score >=9.4

distant
metastasis

E-T-CMF

E-CMF

months

EP 2 553 119 B1

# FIG 21

FIG 22

all patients

E-CMF

E-T-CMF

overall survival

months

patients with score <11.3

E-T-CMF

E-CMF

overall survival

months

stratification

patients with score >=11.3

E-T-CMF

E-CMF

overall survival

months

FIG 23

all patients

overall survival

E-CMF

E-T-CMF

months

patients with score <15.5

overall survival

E-CMF

E-T-CMF

months

stratification

patients with score >=15.5

overall survival

E-T-CMF

E-CMF

months

FIG 24

FIG 25